# EUROPEAN PATENT SPECIFICATION

(11) **EP 4 643 162 B1**
(45) Date of publication and mention of the grant of the patent: **02.12.2026**
(21) Application number: 23855799.5
(22) Date of filing: 28.12.2023
(51) Int. Cl.: G01T 1/20, G01T 1/29

(54) **REFERENCE DETECTOR ARRANGEMENT FOR COMPUTED TOMOGRAPHY IMAGING SYSTEM**
REFERENZDETEKTORANORDNUNG FÜR EIN COMPUTERTOMOGRAPHIE-BILDGEBUNGSSYSTEM
DISPOSITIF DE DETECTEUR DE REFERENCE POUR UN SYSTÈME D'IMAGERIE PAR TOMODENSITOMÉTRIE

(30) Priority: 30.12.2022 US 202263436188 P
(43) Date of publication of application: 05.11.2025
(73) Proprietor: Stryker Corporation, Portage, MI 49002 (US)
(72) Inventor: WILCOX, Kevin, John, Medford, MA 02155 (US); POWELL, Robert, Coughlin, Stow, MA 01775 (US); HILTS, Kenneth, L., Merrimac, MA 01860 (US); WOOLDRIDGE, John, Leominster, MA 01453 (US); HEATH, Nicole, F., Brookline, NH 03033 (US); MAGOVERN, Scott, Chelmsford, MA 01863 (US)
(74) Representative: Röthinger, Rainer
(86) International application number: PCT/US2023/086142
(87) International publication number: WO 2024/145417

(56) References cited:
- CN-A- 101 090 668
- GB-A- 2 187 370
- JP-A- 2016 223 997
- US-A1- 2015 049 857
- US-A1- 2019 282 185
- US-B2- 9 111 379

## Description

### CROSS-REFERENCE TO RELATED APPLICATION(S)

### BACKGROUND

Conventional medical imaging devices, such as computed tomography (CT) and magnetic resonance (MR) imaging devices, are typically realized with fixed or otherwise relatively immobile devices located in a discrete area reserved for imaging that is often far removed from the point-of-care where the devices could be most useful.

For certain procedures, patient-specific imaging data may be acquired intraoperatively using one or more types of imaging systems to help assist the surgeon in visualizing, navigating relative to, and/or treating the anatomy. To this end, navigation systems may cooperate with imaging systems and/or other parts of surgical systems (e.g., surgical tools, instruments, surgical robots, and the like) to track objects relative to a target site of the anatomy.

Computed tomography imaging systems generally use some form of reference detector assembly operatively attached to the x-ray source for measuring a flux of photons generated by the x-ray source. However, there remains a need in the art to maximize x-ray protection of components of the reference detector assembly.

US2019282185 and US9111379B2 disclose an x-ray CT system, comprising: a gantry with a rotor arranged for rotation about an axis; an x-ray source supported on the rotor and configured to generate x-rays; an x-ray detector supported on the rotor; a reference detector assembly operatively attached to the x-ray source for measuring flux of photons generated by the x-ray source, the reference detector assembly including: an x-ray sensitive element configured to generate a reference output in response to x-rays generated by the x-ray source passing through an aperture of a brass housing, a photodiode supported adjacent to the x-ray sensitive element and configured to receive the reference output from the x-ray sensitive element, and a reference detector controller in communication with the photodiode and configured to generate a reference signal based on the reference output from the x-ray sensitive element; and a controller including a memory and a processor coupled to the memory and configured with processor-executable instructions to perform tomographic reconstruction of image data received from the x-ray detector. This reference detector can provide X-ray photon flux and X-ray source temperature to an integrated processor of the detector.

US2015049857 shows an X-ray CT-system with a rotating gantry and reference detectors comprising an X-ray lens, a tungsten X-ray blocking layer, a scintillating array and an array of photodiodes. The tungsten blocking layer operates to reduce the intensity of the X-ray radiation by attenuation. An X-ray lens with openings transmits the X-rays. A reference signal from central apertures of the lens may be used for flux normalisation.

### SUMMARY

The present teachings generally provide for an x-ray CT system according to claim 1.

### BRIEF DESCRIPTION OF THE DRAWINGS

Advantages of the present disclosure will be readily appreciated as the same becomes better understood by reference to the following detailed description when considered in connection with the accompanying drawings.
Figure 1 is a perspective view of an imaging system.
Figure 2 is a cross-sectional schematic illustration of an imaging system that illustrates the rotating and non-rotating portions of the system.
Figure 3 is an enhanced schematic view of a portion of Figure 2.
Figure 4 illustrates a drive mechanism of the imaging device.
Figure 5 illustrates the imaging system performing a helical scan.
Figures 6A and 6B show the imaging system moving between a first position and a second position.
Figure 7 illustrates one example of an x-ray source with a reference detector assembly.
Figure 8A is a perspective view of a first instance of the reference detector assembly.
Figure 8B is an exploded view of the first instance of the reference detector assembly.
Figure 9A is a perspective view of a second instance of the reference detector assembly.
Figures 9B and 9C are exploded views of the second instance of the reference detector assembly.
Figure 10A is a perspective view of a third instance of the reference detector assembly.
Figures 10B and 10C are exploded views of the third instance of the reference detector assembly.
Figure 11A is a perspective view of a fourth instance of the reference detector assembly.
Figures 11B and 11C are exploded views of the fourth instance of the reference detector assembly.
Figure 12A is a perspective view depicting portions of a fifth instance of the reference detector assembly.
Figures 12B, 12C, and 12D are exploded view of the fifth instance of the reference detector assembly.

### DETAILED DESCRIPTION

The various versions of the present disclosure will be described in detail with reference to the accompanying drawings. Wherever possible, the same reference numbers will be used throughout the drawings to refer to the same or corresponding parts. References made to particular examples and implementations are for illustrative purposes and are not intended to limit the scope of the present disclosure.

The present disclosure generally relates to an imaging system 100 (also known as a surgical imaging system). The imaging system 100 may be used for pre-operative planning, intraoperative use, and/or post-operative follow up. The imaging system 100 may function with an x-ray imaging device 10 (and/or other types of imaging devices) to acquire x-ray images (e.g., patient imaging data) of one or more anatomical objects of interest and display the x-ray images to a surgeon or surgery team. For example, the imaging system 100 may take and display an x-ray image of a particular patient P anatomical feature or region (e.g., knee, spine, ankle, foot, neck, hip, arm, leg, rib cage, hand, shoulder, head, the like, and/or combinations thereof). In some examples, the imaging system 100 may function to superimpose an image of surgical instruments 106, 108 over the displayed x-ray image of the anatomical feature, displaying the surgical instruments 106, 108 relative the anatomical feature. The imaging system 100 may function to acquire multiple x-ray images forming a CT scan of a patient P. The imaging system 100 may be configured to automatically correlate a position of an x-ray imaging device 10 with a portion of the x-ray images taken during a scan. The imaging system 100 may register the x-ray images with the position of the x-ray images based on information generated by the navigation system 16 including an optical sensor (e.g., camera units 56 of a localizer 54). In some versions, the imaging system 100 comprises an x-ray imaging device 10 (also referred to as an imager) including a base 20, a gimbal 30, a gantry 40, and a pedestal 50. The gantry 40 is configured to translate along the base 20.

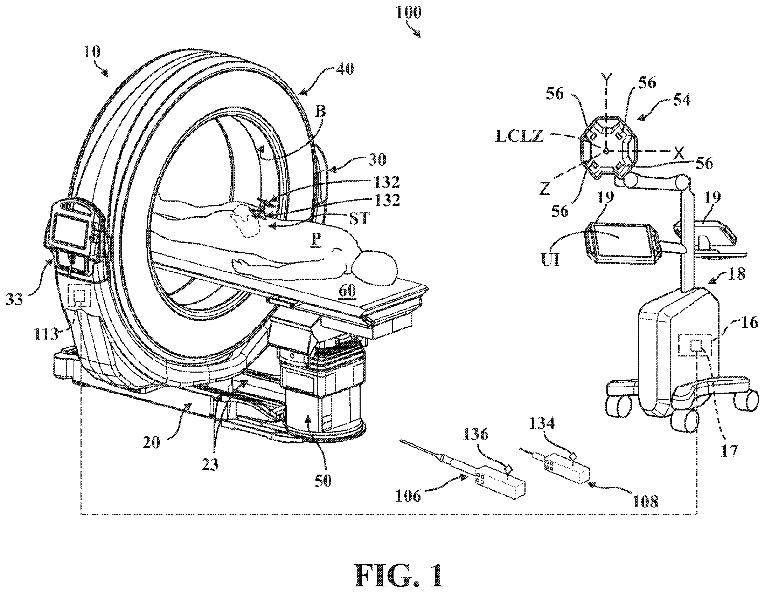

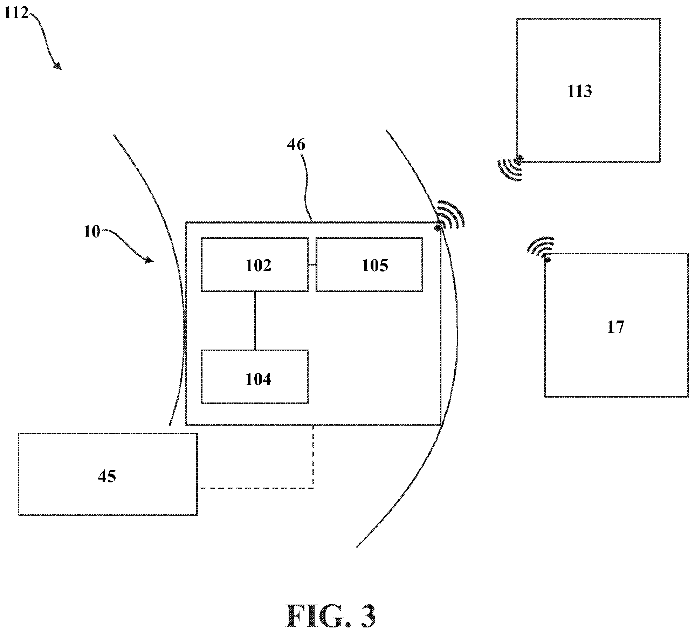

Referring to Figure 1, in some versions, the navigation system 16 may employ a navigation controller 17 that communicates with an imager system controller 113 of the x-ray imaging device 10. The imaging system 100 is configured to collect imaging data, such as, for example x-ray computed tomography (CT) or magnetic resonance imaging (MRI) data, from an object located within a bore B of the gantry 40, in any manner known in the medical imaging field, and to register the collected imaging data in a navigation reference frame of the navigation system 16. As best seen schematically in Figure 3, at least the imager system controller 113, the navigation controller 17, a controller 46 (also referred to as an "on board computer") may for part of the control system 112 of the imaging system 100 as described in greater detail below.

Referring to Figure 1, as noted above, the imaging system 100 may include the navigation system 16. One example of the navigation system 16 is described in U.S. Patent No. 9,008,757, filed on September 24, 2013. The navigation system 16 tracks movement of various objects, such as, for example, portions of the x-ray imaging device 10 (e.g., gantry 40, rotor 41, base 20, pedestal 50, tabletop support 60), one or more surgical instruments 106, 108 or tools, anatomy of a patient P (e.g., the spine or other bone structures, such as one or more vertebra, the pelvis, scapula, or humerus), and/or combinations thereof. The navigation system 16 monitors or otherwise tracks these objects and may gather state information of each object with respect to a (navigation) localizer coordinate system LCLZ. As used herein, the state of an object includes, but is not limited to, data that defines the position and/or orientation of the tracked object (e.g., coordinate systems thereof) or equivalents/derivatives of the position and/or orientation. For example, the state may be a pose of the object, and/or may include linear velocity data, angular velocity data, and the like. In some examples, such as shown in Figure 1, the navigation controller 17 is operatively connected with the control system 112 of the imaging system 100.

The navigation system 16 may employ a mobile cart assembly 18 that houses a navigation controller 17, and/or other types of control units. A navigation user interface UI is in operative communication with the navigation controller 17. The navigation user interface UI includes one or more display devices 19. The navigation system 16 is capable of displaying graphical representations of the relative states of the tracked objects to the user using the one or more display devices 19. The navigation user interface UI further comprises one or more input devices (not shown in detail) to input information into the navigation controller 17 or otherwise to select/control certain aspects of the navigation controller 17. Such input devices include interactive touchscreen displays. However, the input devices may include any one or more of push buttons, pointer, foot switches, a keyboard, a mouse, a microphone (voice-activation), gesture control devices, and the like. In some examples, the user may use buttons located on the surgical instrument 106 (e.g., a pointer) to navigate through icons and menus of the user interfaces UI to make selections, configuring the imaging system 100 and/or advancing through the workflow.

In the illustrated versions, the localizer 54 of the navigation system 16 is coupled to the navigation controller 17. In some versions, the localizer 54 is an optical localizer and includes a camera unit 56. In certain configurations, the localizer 54 may be similar to as is described in U.S. Patent No. 10,959,783 filed April 15, 2016. The localizer 54 may function to monitor and track tracking devices 132, 134, 136 (also referred to as "trackers") that are coupled to or otherwise supported on various tracked objects, such as the x-ray imaging device 10, surgical instruments 106, 108, the patient P, and/or combinations thereof. One suitable localizer 54 is the FP8000 tracking camera manufactured by Stryker Corporation (Kalamazoo, Mich.).

As best shown in Figure 1, the pedestal 50 is adapted to support a tabletop support 60 that can be attached to the pedestal 50 in a cantilevered manner and extend out into the bore B of the gantry 40 to support a patient P or other object being imaged. In some examples, the tabletop support 60 can be partially or entirely removed from the pedestal 50, and the gantry 40 can be rotated relative to the base 20, preferably at least about 90 degrees, from an imaging position to a transport position to facilitate transport and/or storage of the x-ray imaging device 10.

The x-ray imaging device 10 functions to acquire images of the patient P or anatomical features of the patient's P body supported on the tabletop support 60 (or on some other type of patient support). The x-ray imaging device 10 may include a structure with an emitting portion configured to generate x-rays, which is realized as an x-ray source 43 (e.g., one or more x-ray tubes or other types of radiation sources) and an imaging portion realized as an x-ray detector 34 (or some other form of detector). The x-ray imaging device 10 may be configured to have a gantry 40 with a general O-shape. The gantry 40 may include the x-ray source 43 and the x-ray detector 45 located on the opposing portions of the gantry 40. The x-ray source 43 and the x-ray detector 45 may be at a fixed distance from each other. An imaging region (not shown in detail) may be defined in the center of the O-shape, within the bore B, between the x-ray source 43 and the x-ray detector 45. A patient P or a portion of a patient P may be located in the center of the bore B of the gantry 40, between the x-ray source 43 and the x-ray detector 45, so that a specific portion of the patient P may be imaged.

The outer diameter of the gantry 40 can be relatively small, which may facilitate the portability of the x-ray imaging device 10. In one example, the outer diameter of the gantry 40 is less than about 70 inches, such as between about 60 and 68 inches, and in some versions is about 66 inches. The outer circumferential wall of the outer shell 42 may be relatively thin to minimize the outer diameter dimension of the gantry 40. In addition, the interior diameter of the gantry 40, or equivalently the bore B diameter, can be sufficiently large to allow for the widest variety of imaging applications, including enabling different patient supports 60 (e.g., tabletop supports 60) to fit inside the bore B, and to maximize access to a subject located inside the bore B. In some versions, the bore diameter of the gantry 40 is greater than about 38 inches, such as between about 38 and 44 inches, and in some versions can be between about 40 and 50 inches. In one exemplary version, the bore B has a diameter of about 42 inches. The gantry 40 generally has a narrow profile, which may facilitate portability of the x-ray imaging device 10. In some versions, the width of the gantry 40 is less than about 17 inches and can be about 15 inches or less.

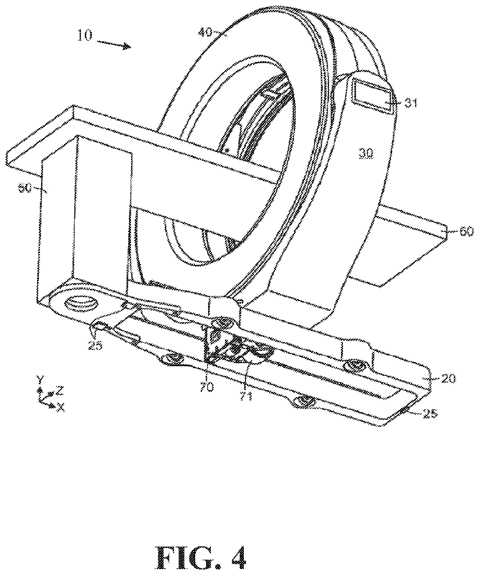

As is best depicted in Figure 4, the x-ray imaging device 10 includes a drive mechanism 70 mounted beneath the gimbal 30 and the gantry 40 and within the base 20. The drive mechanism 70 also comprises a drive wheel 71 that can extend and retract between a first extended position to facilitate transport of the x-ray imaging device 10, and a second retracted position during an image acquisition procedure (e.g., during an imaging scan). The drive mechanism 70 includes a main drive (not shown in detail) that is geared into the drive wheel 71 when the drive wheel 71 is in the first extended position to propel the x-ray imaging device 10 across a floor or other surface, and thus facilitate transport and positioning of the x-ray imaging device 10. In some versions, the drive wheel 71 can be decoupled from the main drive when the drive wheel 71 is in the second retracted position, thus preventing the x-ray imaging device 10 from back-driving the main drive during an imaging procedure. In some versions, the drive mechanism 70 includes one or more sensors (not shown) to track the position of the drive wheel 7, the position of the gimbal 30 and gantry 40, and the like, relative to the base 20 and/or to other components of the x-ray imaging device 10.

As is illustrated in Figure 1, the base 20 is realized as a sturdy, generally rectilinear support structure, and includes a central opening extending lengthwise along the base 20 in which the drive mechanism 70 is positioned. In some examples the bottom of the base 20 includes a plurality of pockets (not shown in detail) that contain casters 21 that are retractable. The casters 21 can be spring-loaded and biased to extend from the bottom of the base 20 when the x-ray imaging device 10 is raised off the ground. When the drive wheel 71 is retracted and the x-ray imaging device 10 is lowered to the ground, the casters 21 are retracted into their respective pockets. In an alternative version, an active drive system, rather than a passive spring-based system, can drive the extension and retraction of the casters in their respective pockets.

The gimbal 30 may be a generally C-shaped support that is mounted to the top surface of base 20 and includes a pair of arms 31, 33 extending up from the base. The arms 31, 33 may be connected to opposite sides of gantry 40 so that the gantry is suspended above base 20 and gimbal 30. In some versions, the gimbal 30 and gantry 40 may rotate together about a first (e.g., vertical) axis with respect to the base 20, and the gantry 40 may tilt about a second (e.g., horizontal) axis with respect to the gimbal 30 and base 20. In some versions, a gimbal drive mechanism (not shown in detail) may be mounted between the gimbal 30 and the base 20 to controllably drive the rotation (i.e., "yaw" motion) of the gimbal 30 and gantry 40 with respect to the base 20. A gimbal drive mechanism may also controllably drive the "tilt" motion of the gantry 40 with respect to the gimbal 30.

The gimbal 30 and gantry 40 may translate with respect to the base 20. The gimbal 30 may include bearing surfaces (not shown in detail) that travel on rails 23, as shown in Figure 1, to provide the translation motion of the gimbal 30 and gantry 40. A scan drive mechanism (not shown in detail) may drive the translation of the gantry 40 and gimbal 30 relative to the base 20, and a main drive mechanism may drive the entire system in a transport mode (e.g., on one or more casters or wheels). In the version of Figure 1, both of these functions are combined in the drive mechanism 70 that is located beneath the gimbal 30. Further details of similar drive mechanisms 70 for x-ray imaging devices 10 are described in U.S. Patent No. 8,753,009, filed February 11, 2011.

The x-ray imaging device 10 generally operates to obtain images of an object located in the bore B of the gantry 40. For example, in the case of an x-ray CT scan, the rotor 41 rotates within the housing of the gantry 40 and about an axis (e.g., a vertical axis with respect to the base 20) while imaging components, including the x-ray source 43 and x-ray detector 45, obtain image data at a variety of scan angles. Generally, the x-ray imaging device 10 obtains image data over relatively short intervals, with a typical scan lasting less than a minute, or sometimes just a few seconds. During these short intervals, however, a number of components, such as the x-ray source 43 and the high-voltage generator 44, require a large amount of power, including, in some versions, up to 32 kW of power.

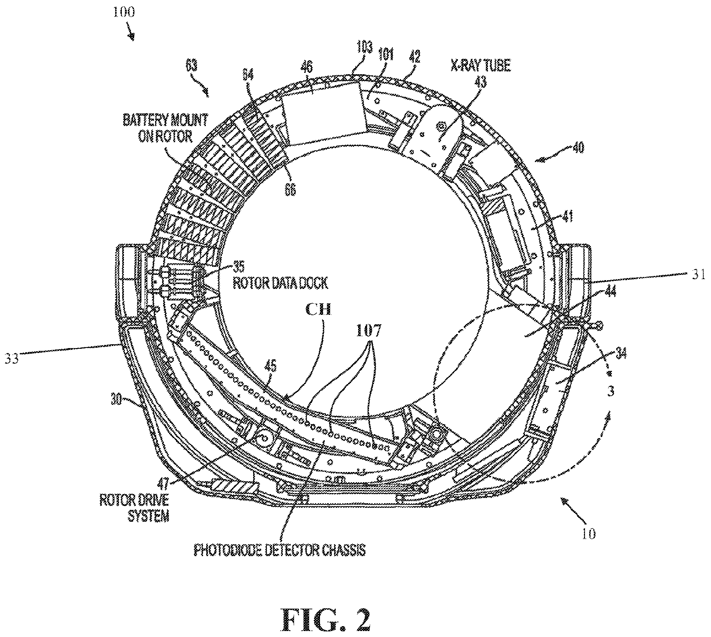

The example illustrated in Figure 2 illustrates a single high voltage generator 44 powering the x-ray source 43. However, it will be understood that in various versions multiple high voltage generators 44 may be provided on the gantry 40, and each x-ray source 43 may have a dedicated high-voltage generator 44. In some versions, one or more high-voltage generators 44 may be provided off of the gantry 40, and high voltage power may be delivered to the x-ray source 43 via a cable or slip ring system (not shown).

The high-voltage generator 44 may be powered by a power source on the gantry 40, such as a battery system 63. As shown in Figure 2, the battery system 63 may be mounted to and rotates with the rotor 41. The battery system 63 may include a plurality of electrochemical cells. The cells may be incorporated into one or more battery packs. The battery system 63 is preferably rechargeable and may be recharged by a charging system (not shown) between imaging operations, such as when the rotor 41 is not rotating. In some versions, the battery system 63 consists of lithium iron phosphate (LiFePO4) cells, though it will be understood that other suitable types of batteries can be utilized.

The battery system 63 provides power to various components of the x-ray imaging device 10. In particular, since the battery system 63 is located on the rotor 41, the battery system 63 may provide power to any component on the rotor 41, even as these components are rotating with respect to the non-rotating portion of the x-ray imaging device 10. Specifically, the battery system 63 is configured to provide the voltages and peak power required by the high-voltage generator 44 and x-ray source 43 (e.g., the x-ray tube) to perform an imaging scan. For example, a battery system 63 may output ~360V or more, which may be stepped up to 120kV at the high-voltage generator 44 to perform an imaging scan. In addition, the battery system 63 may provide power to operate other components, such as an on-board computer or controller 46, the x-ray detector 45, and a drive mechanism 47 for rotating the rotor 41 within the gantry 40. Here, in some versions, the drive mechanism 47 drives the rotation of the rotor 41 around the interior of the gantry 40. The drive mechanism 47 may be controlled by the imager system controller 113 that controls the rotation and precise angular position of the rotor 41 with respect to the gantry 40, such as by using position feedback data from one or more encoder devices (not shown). The drive mechanism 47 may include a motor and gear system mounted to the rotor 41 (see Figure 2; not shown in detail). The motor may drive a gear that may be engage with a mating component on the non-rotating portion of the x-ray imaging device 10 to drive the rotation of the rotor 41. For example, a belt 82 may be rotatably fixed on the non-rotating portion of the x-ray imaging device 10 (e.g., the outer shell of the gantry 40), such as on a circumferential rail. The drive mechanism 47 may engage with the belt 82 to drive the rotation of the rotor 41 within the gantry 40. The drive mechanism 47 may be powered by the battery system 63, may be secured to the rotor 41, and may be positioned behind the x-ray detector 45, as shown in Figure 2. Further details of a similar type of drive mechanisms 47 are described in U.S. Patent No. 9,737,273, filed April 6, 2012.

An on-board computer 46 may be provided on the rotating portion of the system and may be secured to rotor 41 in a suitable location, as shown in Figure 2. Figure 3 is an enhanced schematic view of the on-board computer 46 including processor 102, memory 104, and transmitter/receiver 105. The on-board computer 46 may be connected with one or more external computers and/or controllers 113 of the control system 112 in a wired or wireless link. The on-board computer 46 may be powered by battery system 63. The on-board computer 46 may be any suitable computing device, and may include one or more processors 102 having associated memory 104 that may execute instructions (e.g., software) stored in memory 104, as is known in the art. The on-board computer 46 may perform various control functions for the various components on the rotor 41 and may serve as an interface between components on the rotor 41 and other components of the x-ray imaging device 10. The on-board computer 46 may be configured to receive imaging data collected by the x-ray detector 45. For example, the x-ray detector 45 may stream their image data over a suitable data connection (e.g., wired or wireless) to the on-board computer 46. The on-board computer 46 may store, process and/or transmit the imaging data. For example, the on-board computer 46 may include or may be coupled to a wireless transmitter that may transmit the data to another logical entity, such as to an external workstation and/or to another controller 113 located on the non-rotating portion of the system (e.g., in the gimbal 30). This may enable real-time display of the collected imaging data.

A docking system 35 may be provided for connecting the rotating portion of the x-ray imaging device 10 to the non-rotating portion between imaging scans. The docking system 35 may include a connector for carrying power between the rotating and non-rotating portions. In some versions, the docking system 35 may be used to provide power to the battery system 63 such that the batteries may be charged using power from an external power source (e.g., grid power). The docking system 35 may also include a data connection to allow data signals to pass between the rotating and non-rotating portions. Further details of a suitable docking system are described in U.S. Patent No. 9,737,273, filed April 6, 2012.

During an imaging scan, the rotor 41 rotates around an object positioned within the bore B, while the imaging components such as the x-ray source 43 and x-ray detector 45 operate to obtain imaging data (e.g., raw x-ray projection data) for an object positioned within the bore B of the gantry 40, as is known, for example, in conventional X-ray CT scanners. The collected imaging data may be fed to an on-board computer 46, preferably as the rotor 41 is rotating, for performing x-ray CT reconstruction, as will be described in further detail below.

Various details of examples of an imaging system can be found in the above-referenced U.S. Patent Nos. 8,118,488, filed January 5, 2009, 8,753,009, filed March 9, 2010, 8,770,839, filed March 19, 2010, and 9,737,273, filed April 7, 2011. It will be understood that these examples are provided as illustrative, non-limiting examples of imaging systems suitable for use in the present methods and systems, and that the present systems and methods may be applicable to imaging systems of various types, now known or later developed.

The x-ray detector 45 may include a plurality of x-ray sensitive detector elements, along with associated electronics, which may be enclosed in a housing or detector chassis CH (FIG. 2). In one example, the detector chassis has a width of 7¾ inches, a depth of between about 4-5 inches and a length of about 1 meter or more, such as about 43 inches. The detector chassis CH may be a rigid frame, which may be formed of a metal material, such as aluminum, and which may be formed by a suitable machining technique. The x-ray detector 45 may be mounted to the rotor 41 opposite an x-ray source 43, as is shown in Figure 2. A plurality of x-ray-sensitive detector elements are located in within detector modules 107 provided in the interior of the detector chassis CH so that the detector elements face in the direction of the x-ray source 43. The detector chassis CH may form a protective air- and light-tight shroud around the detector elements, so that unwanted air and light may not contaminate the sensitive components housed within the x-ray detector 45.

In various examples, the individual detector elements may be located on a plurality of detector modules 107. Figure 3 illustrates an array of detector modules 107 arranged within a detector chassis CH of x-ray detector 45. Each individual detector element, which may be for example, a cadmium tungstate (CdWO₄) material coupled to a photodiode, represents a pixel on a detector module 107 with multiple elements. The detector modules 107 may be 2D element array, with for example 512 pixels per module (e.g., 32×16 pixels).

The x-ray detector 45 may include one or more detector modules 107 mounted within the detector chassis CH. The detector module(s) 107 may be arranged along the length of the detector chassis CH to form or approximate a semicircular arc, with the arc center coinciding with the focal spot of detector the x-ray source 43. In one example, the x-ray detector 45 includes thirty-one two-dimensional detector modules 107 positioned along the length of the detector chassis CH, and angled relative to each other to approximate a semicircular arc centered on the focal spot of the x-ray source. Each detector module 107 may be positioned such that the detector module 107 surface is normal to a ray extending from the x-ray focal spot to the center pixel of the detector module 107.

It will be understood that the x-ray detector 45 may include any number of detector modules 107 along the length of the detector. As shown in FIG. 3, for example, a detector may include "m" modules 107, where "m" may be any integer greater than or equal to 1. Further, each detector module 107 may include an arbitrary number of individual elements (pixels) in the module. Larger and/or a greater number of detector modules 107 may allow a larger diameter "back projection" area around the isocenter of the imaging system, and thus may allow a larger cross-section of the object to be reconstructed.

Each of the detector modules 107 may include an array of photosensitive elements which may be electrically and optionally physically coupled to a circuit board that may include one or more electronic components. In some examples, the detector modules 107 may plug into a circuit board using a suitable electronic connection such as described in United States Patent No. 9,111,379, filed June 28, 2012. The circuit board may be configured to couple the raw analog signals from each detector element in the array into an analog-to-digital converter (herein referred to as A/D converter) for converting the signal to a digital signal. In some examples, the circuit board includes several A/D converters. Each detector element may provide its analog signal over a separate channel into the A/D converters. For example, where the array includes 512 pixels, four 128-channel A/D converters may be provided to convert the analog signal from each element into a digital signal.

The circuit board may include a processor, which may be, for example, an FPGA. The processor may receive the digital image data from the A/D converters, which may be in a digital video format, such as LVDS, and may be programmed to assemble the data into a single image. The processor may be configured to convert the image data to a different digital video format, such as Camera Link. In examples, the processor may convert the image data into another suitable format, such as gigabit Ethernet. The processor may also be programmed to receive image data from one or more other detector modules 107, which may be combined with the image data from the A/D converter(s) and passed off of the detector module 107 in a daisy-chain configuration. In some examples, the processor may receive and transmit the image data in a Camera Link digital video format.

It will be understood that the number of modules (m) in the x-ray detector 45 may vary, and modules may be added or removed as needed. In various examples, changing the number and/or types of detector modules does not require a new or modified "backplane" electronics board, for example. Also, the clock signal (e.g., a Camera Link clock signal) may be variable to provide more or less image frames per second.

As shown in the examples of Figures 2 and 3, the detector modules 107 of the x-ray detector 45 may be electronically connected to the on-board computer 46 which may be located on the rotatable portion 101 of the system (e.g., mounted to the rotor 41). The processor 102 of the on-board computer 46 may be configured to perform tomographic reconstruction of image data that is sent to the on-board computer 46 from the detector modules 107. The on-board computer 46 may wirelessly transmit tomographic reconstruction data (e.g., 3D images of the object) to the imager system controller 113, which may be another computer, such as an external workstation, or a separate computer on the imaging system 100 (e.g., a computer on a gimbal that supports the gantry). In other examples, the on-board computer 46 may transmit tomographic reconstruction data to another entity using a wired link (e.g., via a slip ring or cable connection to the non-rotating portion 103, or via a data dock to the non-rotating portion 103 in between scans). In some examples, it will be understood that in addition to on-board computer 46 and x-ray detector 45, the processor 102 for performing the reconstruction may be at any location on the rotating portion 101 (e.g., rotor 41).

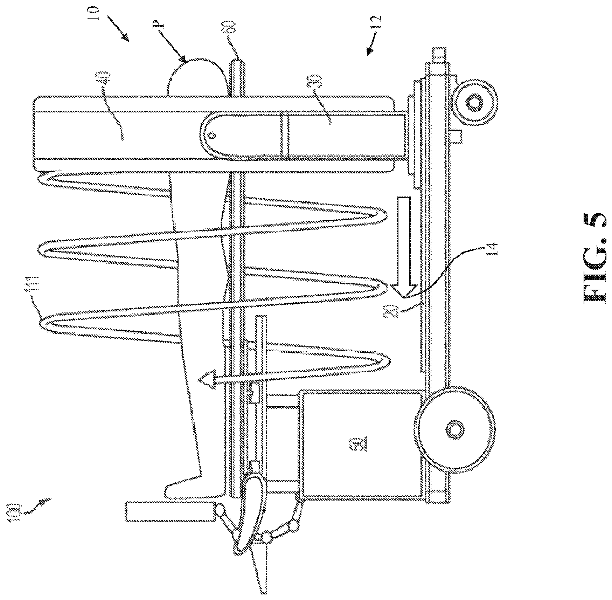

The imaging system 100 may be used to perform cone beam CT imaging. The rotor 41 may rotate within the gantry 40 while the x-ray detector 45 obtain images. The image data may then be reconstructed using a tomographic algorithm as is known in the art to obtain a 3D reconstructed image of the object. In some examples, the x-ray detector 45 may obtain images which may be combined for the reconstruction. Figure 5 illustrates an example helical scan path of the gantry 40 and the rotation of the x-ray source 43 and x-ray detector 45 on rotor 41 between a first position 12 and a second position 14. In some examples, the rotor 41 may only need to rotate a portion of the distance that would normally be required (e.g., a 90° rotation of the rotor 41 may enable the detector to scan 180° of the object, a 270° rotation of the rotor 41 enables a full 360° scan of the object). In some versions, the gantry 40 and gimbal 30 may be translated along rails 23 during cone beam CT imaging to provide a helical cone beam CT scan (Figure 5). In some versions, a helical cone beam scan may be coordinated with the injection of a contrast agent to provide a three-dimensional arterial roadmap image.

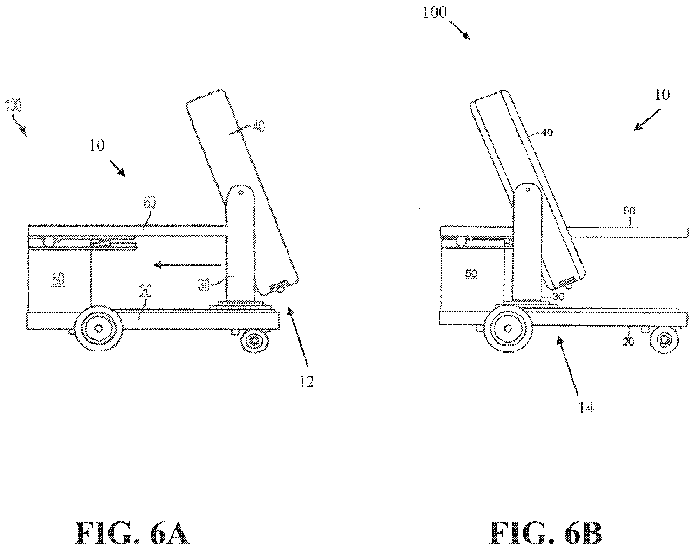

As mentioned above, the gantry 40 may be moved between a plurality of positions and is configured to translate and/or tilt about the base 20 of the x-ray imaging device 10. The gantry 40 is configured to move relative the base 20 to capture x-ray images of a patient P or anatomical feature of interest (e.g., a target site ST), at one or more angled relative to a patient P or particular anatomical feature, raise, lower, repositioned, or a combination thereof. During movement, the x-ray source 43 and the x-ray detector 45 maintain a fixed relationship, keeping the same distance on the opposite ends of the gantry 40. As best seen in Figures 6A and 6B, the gantry 40 is configured to move between a first position 12 and second position 14 and may include a plurality of intermediate positions (e.g., transistor and/or intermittent movement) between the first position 12 and the second position 14.

In various examples, the imaging system 100 may be used to pass "scout" scan data from the rotor 41 in real-time. Figures 6A and 6B illustrate the gantry 40 translating along the base 20 between positions 12, 14. In Figure 6A, the gantry 40 is in a first position 12 and Figure 6B illustrates the gantry 40 in the second position 14 after the gantry 40 has translated along the base 20. A scout scan may be performed while the rotor 41 is not rotating to provide a series of scan lines of the patient (e.g., as the source and detector translate along the patient axis), which may be useful, for example, in choosing a subregion to perform a full 3D scan. The scan lines may be provided from the x-ray detector 45 to processor 102, as described above, which may transmit the scan lines in real time to an external entity (such as a workstation or other computer) for displaying a 2D image of the patient in real-time. During a scout scan, the x-ray beam from the x-ray source 43 may only require a fraction of the size of the x-ray beam required for a full helical scan since the scout scan a preview of the surgical area.

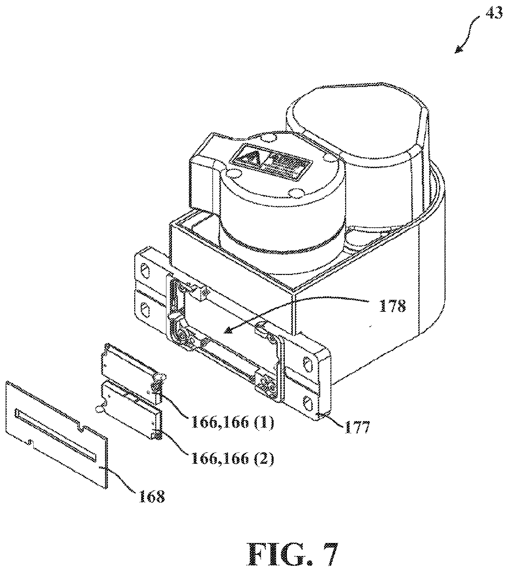

Figure 7 shows one example of an x-ray source 43 with a collimator 168 and one or more reference detector assemblies 166 operatively attached. The collimator 168 is stationary relative to the x-ray source 43. The collimator 168 is connected to a mount 177 locating the collimator 168 axially with the x-ray beam outlet port 178. Reference detector assemblies 166 are disposed between the mount 177 and the collimator 168. In this example, the x-ray beam produced by the x-ray source 43 will fully illuminate the x-ray detector 45 when an image is taken. The reference detector assemblies 166 are configured to measure a flux of photons generated by the x-ray source 43.

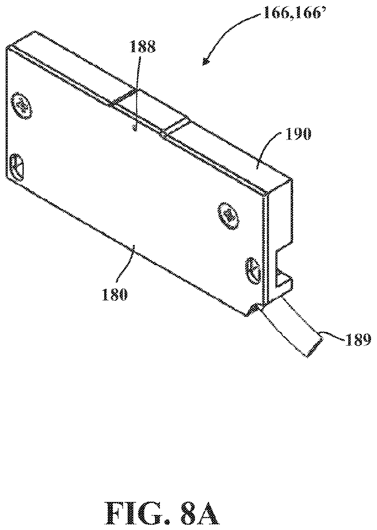

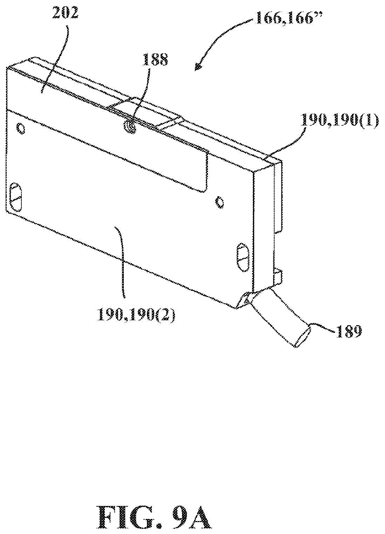

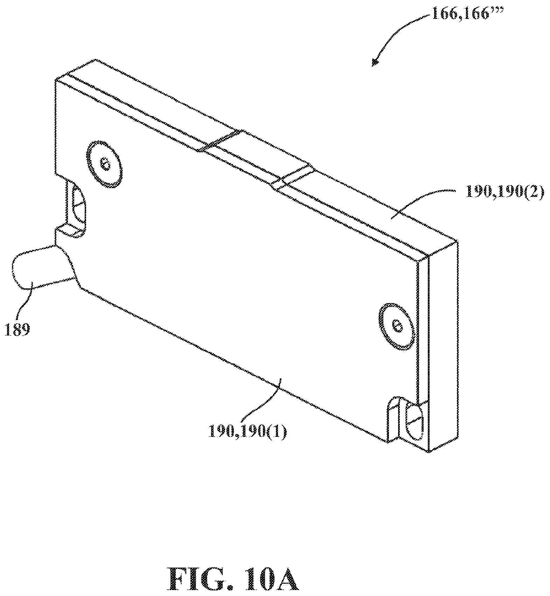

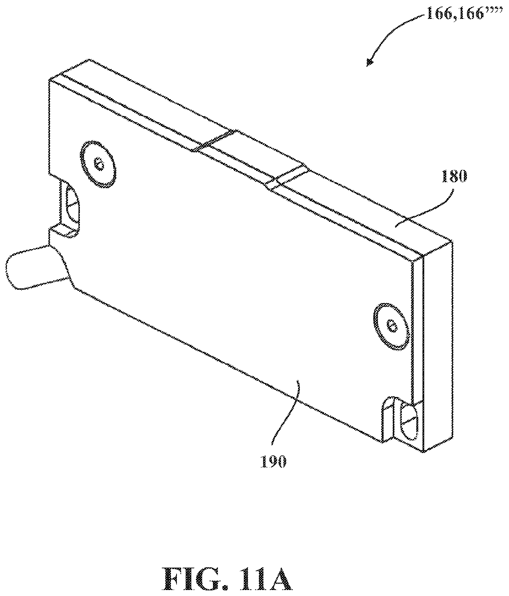

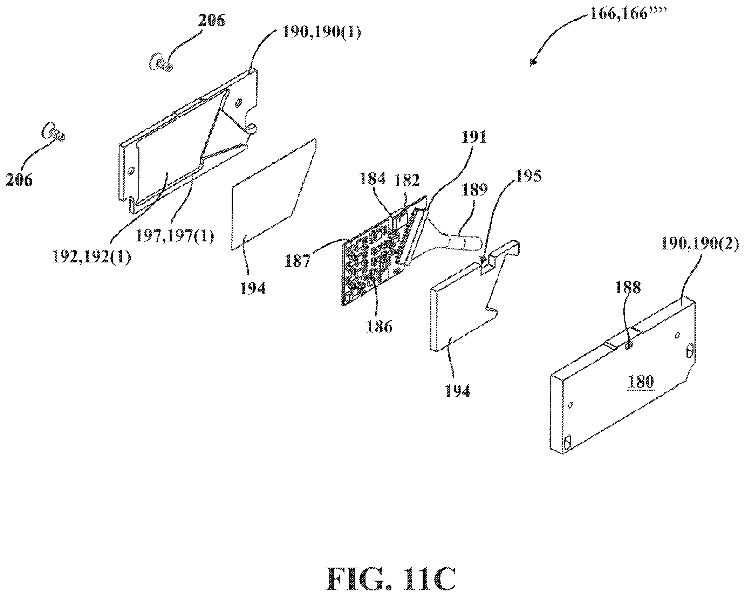

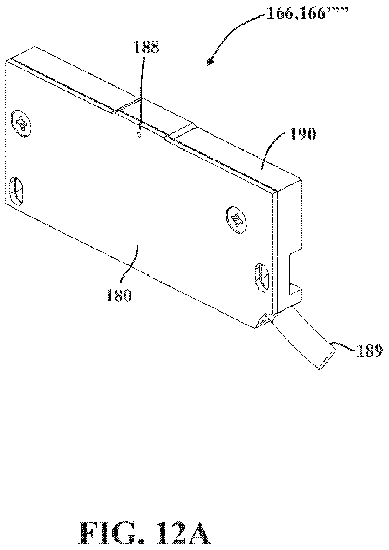

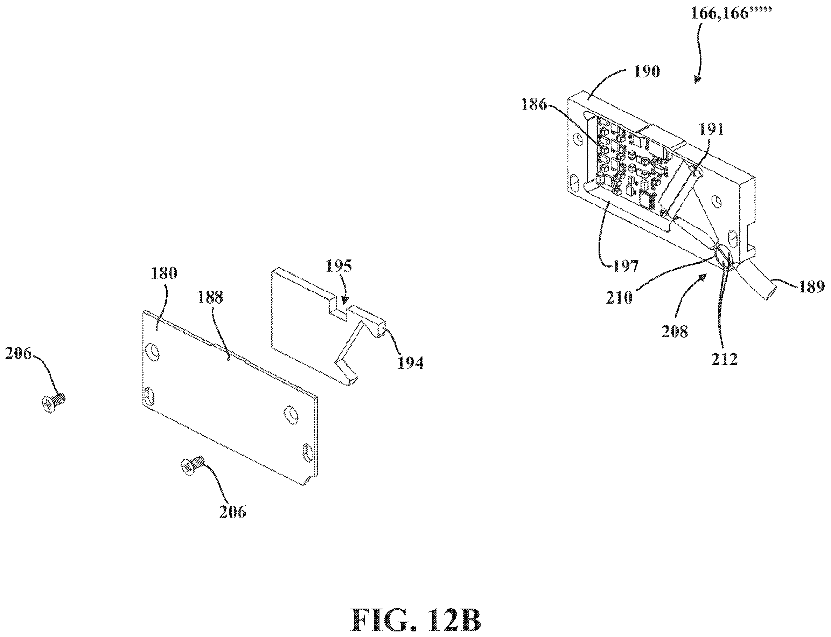

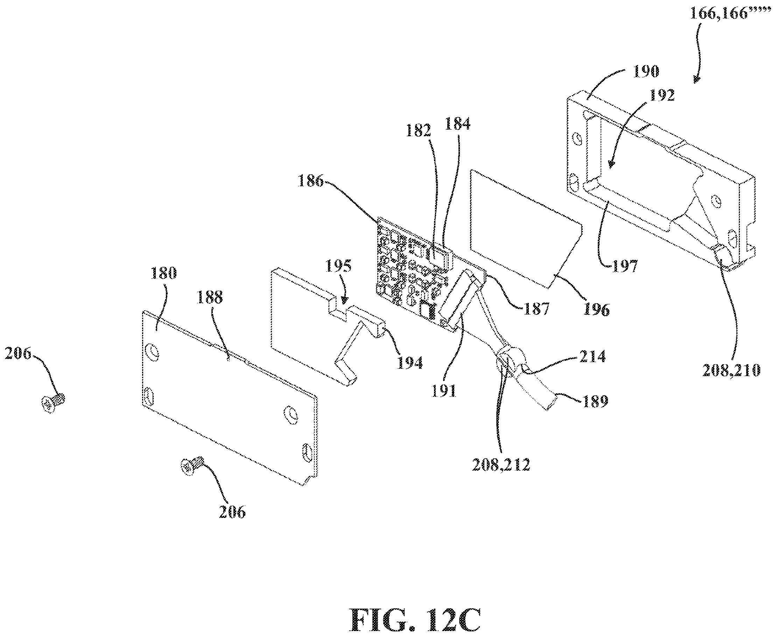

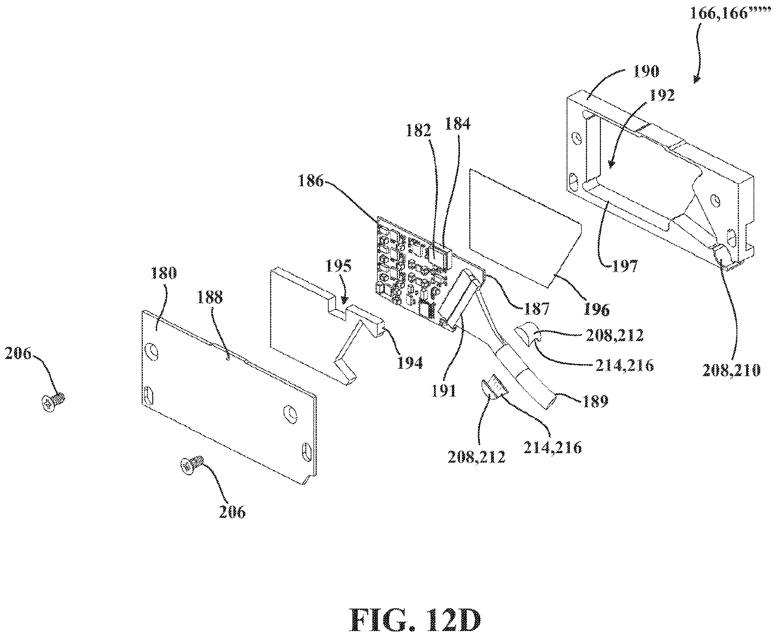

Herein, various instances of the reference detector assembly 166 are shown. A first instance of the reference detector assembly 166' is shown in Figures 8A-8C, a second instance of the reference detector assembly 166" is shown in Figures 9A-9C, a third instance of the reference detector assembly 166‴ is shown in Figures 10A-10C, a fourth instance of the reference detector assembly 166ʺʺ is shown in Figures 11A-11C, and a fifth instance of the reference detector assembly 166ʺ‴ is shown in Figures 12A-12D.

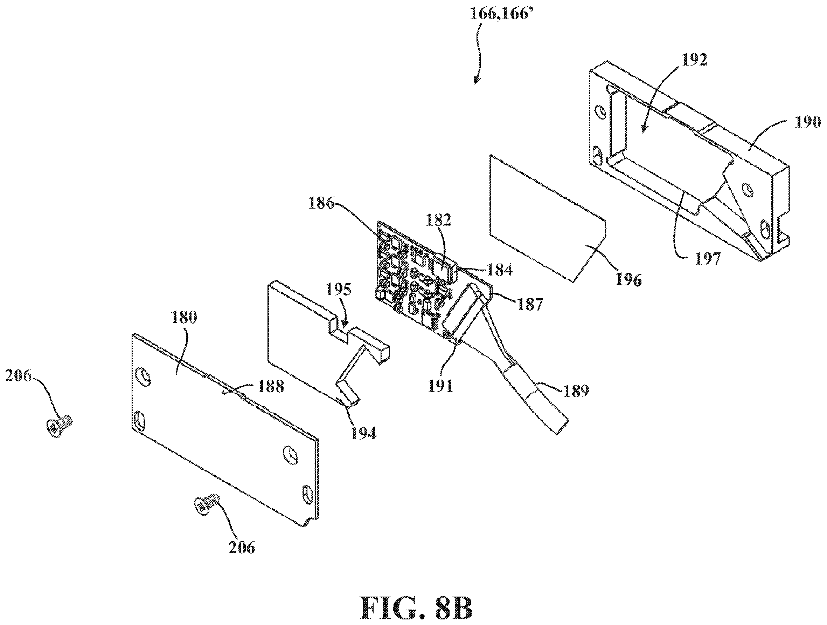

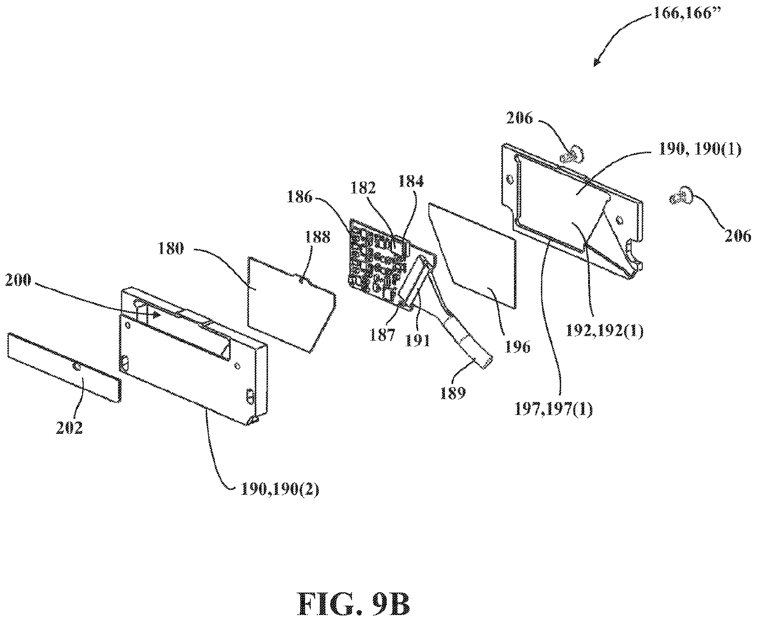

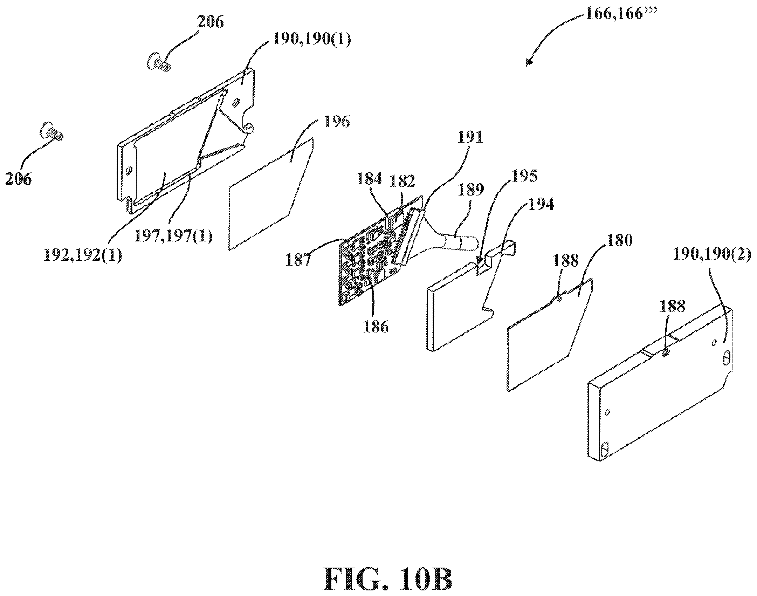

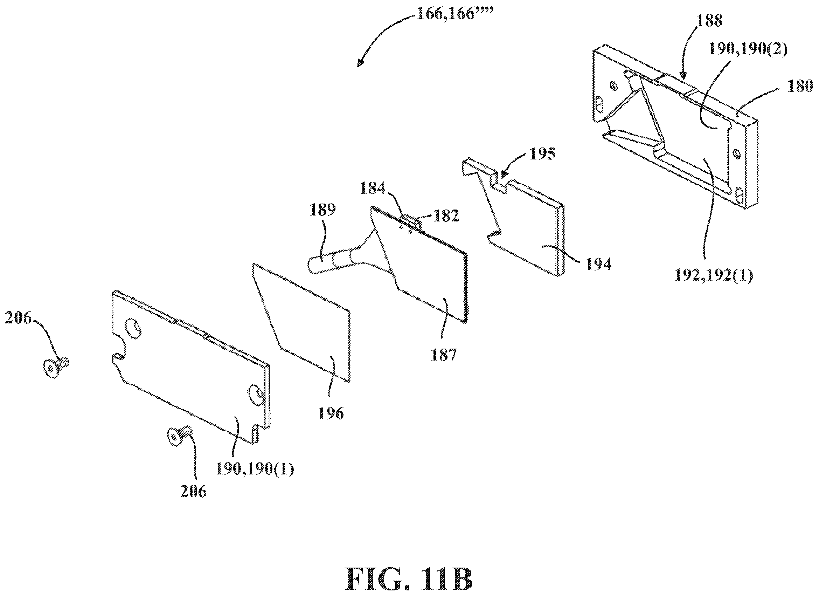

Figure 8B illustrates components of the first instance of the reference detector assembly 166', Figures 9B-9C illustrate components of the second instance of the reference detector assembly 166", Figures 10B-10C illustrate components of the third instance of the reference detector assembly 166‴, Figures 11B-11C illustrate components of the fourth instance of the reference detector assembly 166ʺʺ, and Figures 12A-12D illustrate components of the fifth instance of the reference detector assembly 166ʺ‴. As shown, each of the first, second, third, fourth, and fifth instances of the reference detector assembly 166', 166", 166‴, 166ʺʺ, 166ʺ‴ includes a tungsten shield 180, an x-ray sensitive element 182, a photodiode 184, and a reference detector controller 186. Additionally, as shown, the photodiode 184 and the reference detector controller 186 may be supported by a reference detector board 187. The reference detector board 187 may be coupled to a harness 189 (e.g., a shielded cable supporting one or more wires) via a connector 191.

The tungsten shield 180 is a component formed of tungsten that is configured to shield components of the reference detector assembly 166 from x-rays generated by the x-ray source 43. Additionally, as shown in Figures 8B, 9B-9C, 10B-10C, and 11B-11C, the tungsten shield 180 defines an aperture 188 configured to allow passage of x-rays generated by the x-ray source 43 therethrough. In this way, the tungsten shield 180 shields components of the reference detector assembly 166 from x-rays generated by the x-ray source 43, while permitting the passage of rays generated by the x-ray source 43 through the aperture 188.

The x-ray sensitive element 182 may be any component configured to receive x-rays and generate a reference output. For example, in some instances, the x-ray sensitive element 182 may be a crystal scintillator and the reference output generated by the x-ray sensitive element may be visible light.

The photodiode 184 may be any component configured to receive the reference output (e.g. visible light) from the x-ray sensitive element 182. For example, the photodiode 184 may be PN photodiode, a PIN photodiode, a Schottky type photodiode, or an Avalanche photodiode. In instances where the x-ray sensitive element 182 generates a reference output that is not visible light, the photodiode 184 may instead be replaced with a component capable of receiving the reference output that is not visible light.

The reference detector controller 186 may be any component configured to be in communication with the photodiode 184 such that the reference detector controller 186 may generate a reference signal based on the reference output generated by the x-ray sensitive element 182.

The reference detector assembly 166 is operatively attached to the x-ray source 43 for measuring flux of photons generated by the x-ray source 43. In order to measure the flux of photos generated by the x-ray source 43, the reference detector assembly 166 allows passage of the x-rays generated by the x-ray source 43 therethrough. Specifically, the aperture 188 of the tungsten shield 180 permits passage of the x-rays generated by the x-ray source 43 therethrough. The x-ray sensitive element 182 is supported adjacent to the aperture 188 and configured to receive the x-rays generated by the x-ray source 43 that pass through the aperture 188. The x-ray sensitive element 182 then generates a reference output in response to x-rays generated by the x-ray source 43 that pass through the aperture 188. The photodiode 184 is supported adjacent to the x-ray sensitive element 182 and configured to receive the reference output from the x-ray sensitive element 182. For example, in instances where the x-ray sensitive element 182 is a crystal scintillator and the reference output generated by the x-ray sensitive element is visible light, the photodiode 184 may receive the reference output by sensing the visible light outputted by the x-ray sensitive element 182. Once the photodiode 184 receives the reference output from the x-ray sensitive element 182, the reference detector controller 186, which is in communication with the photodiode, generates a reference signal based on the reference output from the x-ray sensitive element 182. The reference signal generated by the reference detector controller 186 corresponds to the reference output and, furthermore, to the flux of photons generated by the x-ray source 43.

The imager system controller 113 may be configured with processor-executable instructions to perform tomographic reconstruction of image data received from the x-ray detector 45 and normalized based on the reference signal received from the reference detector controller 186. In this way, the imager system controller 113 may be configured to perform tomographic reconstruction of image data received from the x-ray detector 45 based on the flux of photons measured by the reference detector assembly 166.

In some instances, such as the instance of Figure 7, where the x-ray source 43 may be operatively attached to more than one reference detector assembly 166, the imager system controller 113 may perform tomographic reconstruction with image data received from the x-ray detector 45 normalized based on one or more reference signals generated by the reference detector controllers 186 of the one or more reference detector assemblies 166. For example, in the instance of Figure 7, a first reference detector assembly 166(1) and a second reference detector assembly 166(2) are operatively coupled to the x-ray source 43 (it should be understood that the first reference detector assembly 166(1) and the second reference detector assembly 166(2) may include any component of any reference detector assembly 166 described herein). The imager system controller 113 may be configured to perform tomographic reconstruction with image data received from the x-ray detector 45 normalized based on the reference signal generated by the reference detector controller 186 of the first reference detector assembly 166(1) and/or the reference signal generated by the reference detector controller 186 of the second reference detector assembly 166(2). For instance, the imager system controller 113 may be configured to perform tomographic reconstruction with image data received from the x-ray detector 45 normalized based on only the reference signal generated by the reference detector controller 186 of the first reference detector assembly 166(1), based on only the reference signal generated by the reference detector controller 186 of the second reference detector assembly 166(2), or based on an average of the reference signal generated by the reference detector controller 186 of the first reference detector assembly 166(1) and the reference signal generated by the reference detector controller 186 of the second reference detector assembly 166(2).

In some instances, the reference detector assembly 166 may include additional components. For example, the first, third, and fourth instances of the reference detector assembly 166', 166‴, 166ʺʺ each include an insulator 194. The insulator 194 may be configured to insulate components of the reference detector assembly 166 from x-rays generated by the x-ray source 43. As another example, the first, second, third, fourth, and fifth instances of the reference detector assembly 166', 166", 166‴, 166ʺʺ, 166ʺ‴ each include a heat transfer pad 196. The heat transfer pad 196 may be configured to transfer heat away from the reference detector board 187. In this way, the heat transfer pad 196 prevents the reference detector board 187 from overheating through use or through exposure to x-rays generated by the x-ray source 43.

The reference detector assembly 166 may also include a temperature sensor, such as a resistance temperature detector (RTD) that may generate an electronic signal indicative of the temperature within the x-ray source 43. The temperature signal may be a digital signal that may be embedded within the image data stream that is sent to the processor 102 for tomographic reconstruction in the manner described above for the reference signal.

The reference detector assembly 166 may include a shielding enclosure 190 configured to shield components of the reference detector assembly from x-rays generated by the x-ray source 43. Additionally, the shielding enclosure 190 may be configured to house the components of the reference detector assembly therein. The shielding enclosure 190 may define an interior 192, wherein components of the reference detector assembly 166 may be disposed. For example, in the second and third instances of the reference detector assembly 166", 166‴, the shielding enclosure 190 includes a first shielding enclosure plate 190(1) defining a first interior 192(1) and a second shielding enclosure plate 190(2) defining a second interior 192(2). In the second and third instances of the reference detector assembly 166", 166‴, when the first shielding enclosure plate 190(1) is operatively attached to the second shielding enclosure plate 190(2) (as shown in Figures 9A and 10A, respectfully), the first interior 192(1) and the second interior 192(2) cooperate to form the interior 192. As shown in Figure 9B-9C, and 10B-10C, the tungsten shield 180 and the reference detector board 187 may be supported between the first and second shielding enclosure plates 190(1), 190(2) such that, when the first shielding enclosure plate 190(1) is operatively attached to the second shielding enclosure plate 190(2) using screws 206, the tungsten shield 180, the x-ray sensitive element 182, the photodiode 184, and the reference detector controller 186 are disposed within the interior 192.

The shielding enclosure 190 may define a seat 197 shaped to receive components of the reference detector assembly 166. For example, referring to Figure 8B, the shielding enclosure 190 defines a seat 197. The seat 197 is shaped to receive the heat transfer pad 196, the reference detector board 187, and the heat transfer pad 196.

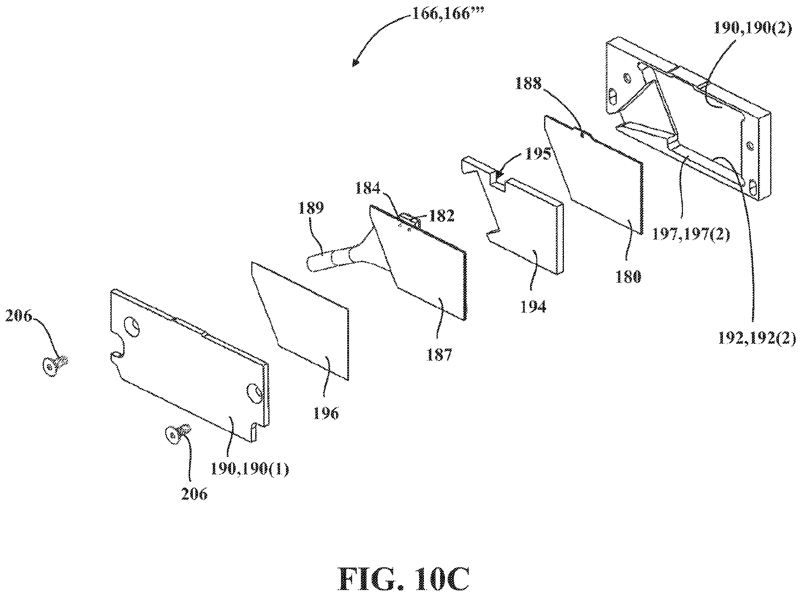

In instances where the reference detector assembly 166 includes a first shielding enclosure plate 190(1) and a second shielding enclosure plate 190(2), one or more of the first shielding enclosure plate 190(1) and the second shielding enclosure plate 190(2) may define the seat 197 shaped to receive components of the reference detector assembly 166. For example, referring to the third instance of the reference detector assembly 166‴ shown in Figures 10B and 10C, the first seat 197(1) and the second seat 197(2) cooperate to form the seat 197 when the first shielding enclosure plate 190(1) is operatively attached to the second shielding enclosure plate 190(2). The seat 197 of the third instance of the reference detector assembly 166‴ is shaped to receive the tungsten shield 180, the reference detector board 187, the insulator 194, and the heat transfer pad 196 within the interior 192.

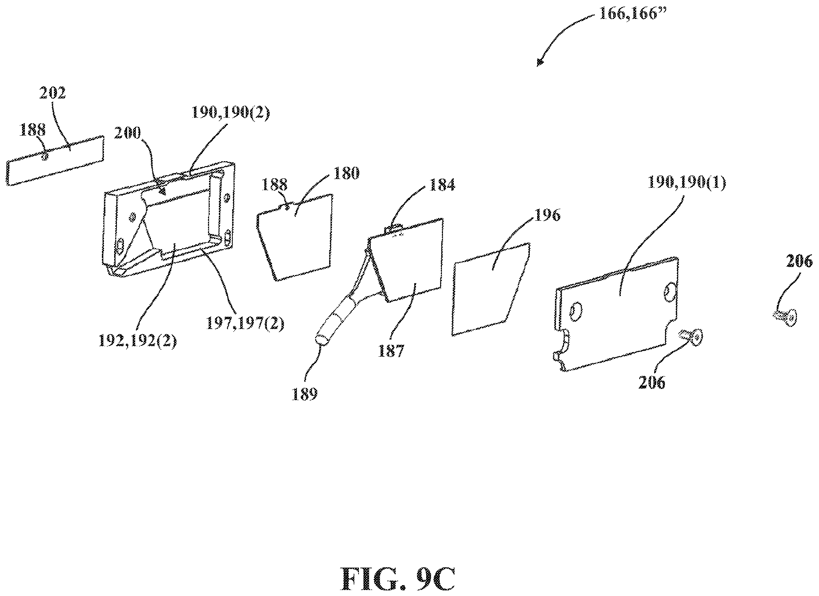

The shielding enclosure 190 may define a window 200 configured to allow passage of the x-rays generated by the x-ray source 43 therethrough. For example, as shown in Figures 9B and 9C, the second shielding enclosure plate 190(2) of the second instance of the reference detector assembly 166'' includes a window 200. The window 200 of reference detector assembly 166'' permits x-rays generated by the x-ray source 43 to pass therethrough and through the aperture 188 towards the x-ray sensitive element 182. It should be noted that, in other instances, the first shielding enclosure plate 190(1) may instead define the window 200. In other instances, both the first and second shielding enclosure plates 190(1), 190(2) may each define a window 200. Additionally, in instances where the shielding enclosure 190 does not include a first and second shielding enclosure plate, such as in the first instance of the reference detector assembly 166' shown in Figure 8B, the shielding enclosure 190 may define a window 200.

In instances where the shielding enclosure 190 defines a window 200, the reference detector assembly 166 may include an auxiliary tungsten shield 202. The auxiliary tungsten shield 202 may define an auxiliary aperture 204 configured to permit the passage of x-rays generated by the x-ray source 43 therethrough. As shown in Figure 9B, the auxiliary tungsten shield 202 may be secured to the window 200 with the auxiliary aperture 204 in alignment with the aperture 188 of the tungsten shield 180 to permit x-rays generated by the x-ray source 43 to pass through the auxiliary aperture 204 and through the aperture 188 towards the x-ray sensitive element 182.

The x-ray sensitive element 182 may be arranged within the interior 192 to optimize reception of x-rays generated by the x-ray source 43 that pass through the aperture 188 of the tungsten shield 180 by the x-ray sensitive element 182. For example, as shown in Figure 8B, Figures 9B-9C, Figures 10B-10C, and Figures 11B-11C, the reference detector board 187 may be disposed within the interior 192 and supporting the photodiode 184, with the x-ray sensitive element 182 arranged between the tungsten shield 180 and the reference detector board 187. Specifically, the x-ray sensitive element 182 may be aligned with and proximate to the aperture 188 of the tungsten shield 180 to optimize reception of x-rays generated by the x-ray source 43 that pass through the aperture 188 of the tungsten shield 180 by the x-ray sensitive element 182.

The reference detector controller 186 may be arranged within the interior 192 to prevent the reference detector controller 186 from receiving x-rays generated by the x-ray source 43, such that the reference detector controller 186 is not damaged by x-rays generated by the x-ray source 43. For example, as shown in Figure 8B, Figures 9B-9C, Figures 10B-10C, and Figures 11B-11C, the reference detector controller 186 is supported on the reference detector board at a location spaced from the aperture 188. Specifically, the reference detector controller 186 (and components thereof) may be vertically and/or horizontally spaced from the aperture 188 such that x-rays generated by the x-ray source 43 that pass through the aperture 188 are not received by the reference detector controller 186.

The insulator 194 may be arranged within the interior 192 to optimize insulation of components of the reference detector assembly 166 from x-rays generated by the x-ray source 43. For instance, referring to Figure 8B, the insulator 194 may be supported within the interior 192 adjacent to the reference detector board 187. Specifically, in the first instance of the reference detector assembly 166', the insulator 194 may be arranged between the reference detector board 187 and the tungsten shield 180. In this way, the insulator 194 insulates the reference detector controller 186 from x-rays generated by the x-ray source 43 that pass through the aperture 188 of the tungsten shield 180. Notably, the insulator 194 may include a cutout 195 such that the insulator 194 does not insulate the x-ray sensitive element 182 from receiving x-rays generated by the x-ray source 43 that pass through the aperture 188.

The heat transfer pad 196 may be arranged within the interior 192 to optimize heat transfer away from components of the reference detector assembly 166. For instance, referring to Figure 8B, the heat transfer pad 196 may be supported within the interior 192 adjacent to the reference detector board 187. Specifically, in the first instance of the reference detector assembly 166', the heat transfer pad 196 may be arranged between the reference detector board 187 and the shielding enclosure 190. In this way, the heat transfer pad 196 transfers heat away from the reference detector board 187 and toward the shielding enclosure 190. In this way, the heat transfer pad 196 may transfer heat away from the reference detector assembly 166 as a whole.

**In** some instances, the tungsten shield 180 may not be disposed within the interior 192. Instead, in such instances, the tungsten shield 180 may be operatively attached to the shielding enclosure 190. In such instances, the tungsten shield 180 and the shielding enclosure 190 cooperate to house components of the reference detector assembly 166 within the interior 192 of the shielding enclosure 190. For example, in the first instance of the reference detector assembly 166' shown in Figure 8A, the tungsten shield 180 is operatively attached to the shielding enclosure 190 using screws 206. As such, in the first instance of the reference detector assembly 166', the tungsten shield 180 and the shielding enclosure 190 cooperate to house the reference detector board 187 and, therefore, the x-ray sensitive element 182, the photodiode 184, and the reference detector controller 186 within the interior 192.

In instances where the tungsten shield 180 is not disposed within the interior 192, the tungsten shield 180 may additionally, or alternatively, define the interior 192. For example, referring to the fourth instance of the reference detector assembly 166ʺʺ shown in Figure 11A, the tungsten shield 180 is operatively attached to the shielding enclosure 190. As shown in Figures 11A and 11B, the tungsten shield 180 defines a first interior 192(1) and the shielding enclosure 190 defines a second interior 192(2). When the tungsten shield 180 is operatively attached to the shielding enclosure 190, the first interior 192(1) of the tungsten shield 180 and the second interior 192(2) cooperate to form the interior 192. In other instances, the tungsten shield 180 may define the entirety of the interior 192.

Components of the reference detector assembly 166 described herein may be formed of any suitable material. For example, although the tungsten shield 180 has been described herein as being formed of tungsten, in other contemplated instances, not encompassed by the appended claims, the tungsten shield 180 may be formed of any material suitable for shielding components of the reference detector assembly 166 from x-rays generated by the x-ray source 43. Similarly, the shielding enclosure 190 may be formed from any material suitable for shielding components of the reference detector assembly 166 housed within the shielding enclosure 190 from x-rays generated by the x-ray source 43. In one instance, the shielding enclosure 190 may be formed from leaded bronze. In another instance, such as the fourth instance of the reference detector assembly 166ʺʺ shown in Figures 11A-11C, the shielding enclosure 190 may be formed from tungsten. In such an instance, the shielding enclosure 190 not only houses components of the reference detector assembly 166 but may also serve as the tungsten shield 180. For example, in the fourth instance of the reference detector assembly 166ʺʺ shown in Figures 11A-11C, the shielding enclosure 190 includes a first shielding enclosure plate 190(1) and a second shielding enclosure plate 190(2). The first shielding enclosure plate 190(1) may be formed of tungsten and may also serve as the tungsten shield 180.

Components of the reference detector assembly 166 described herein may be manufactured using any suitable manufacturing process. For example, the shielding enclosure 190 and the tungsten shield 180 may be formed using an additive manufacturing process. In one such instance, the shielding enclosure 190 and/or the tungsten shield 180 may be formed using an additive manufacturing process such as selective laser melting and/or laser sintering.

Components of the reference detector assembly 166 may include any suitable shape and size. For example, the reference detector assembly 166 and components therein may be sized and shaped such that the reference detector assembly 166 may be operatively attached to the x-ray source 43 and may be disposed between the mount 177 and the collimator 168 (shown in Figure 7). Furthermore, the reference detector assembly 166 and components therein may be sized and shaped such that a greater or fewer number of reference detector assemblies 166 may be operatively attached to the x-ray source 43 and may be disposed between the mount 177 and the collimator 168 (shown in Figure 7). As another example, the aperture 188 of the tungsten shield 180 and the aperture 204 of the auxiliary tungsten shield may be sized to allow a greater or lesser number of x-rays generated by the x-ray source 43 therethrough.

As noted above, the x-ray imaging device 10 includes the x-ray source 43, such as an x-ray tube, that is configured to direct radiation, including collimated x-ray radiation, onto the x-ray detector 45. The x-ray source 43 may be configured to generate a fan beam of x-rays. The x-ray source 43 may include a beam steering mechanism that may alter the direction of the output beam by a particular angle, such as 90° or more. In some examples, the x-ray imaging device 10 may include two or more radiation sources and two or more detectors such that at least a portion of the output radiation beam is alternately centered on a first detector and a second detector, which may be spaced by 90° to provide bi-planar imaging, such as described in U.S. Patent 9,526,461, filed June 25, 2013. Additionally, in instances, such as the instance of Figure 7, where more than one reference detector assembly 166 is operatively attached to the x-ray source 43, the x-ray source 43 may be configured to generate x-rays based at least partially on one or more of the reference signals generated by the reference detector controller 186 of the more than one reference detector assemblies 166. Specifically, in the instance of Figure 7, the x-ray source may be configured to generate x-rays based at least partially on the reference signal generated by the reference detector controller 186 of the first reference detector assembly 166(1) and/or based at least partially on the reference signal generated by the reference detector controller 186 of the second reference detector assembly 166(2).

In some instances, a retainer assembly 208 may be provided to facilitate limiting relative movement between the harness 189 and one or more portions of the shielding enclosure 190. For example, referring to the fifth instance of the reference detector assembly 166ʺ‴ shown in Figures 12A-12D, the shielding enclosure 190 defines a relief 210 arranged adjacent to the seat 197 which is shaped to receive a portion of the harness 189 between a pair of keepers 212. Here, the keepers 212 are each shaped to engage and compress against the portion of the harness 189, and are shaped to be received within the relief 212. In the illustrated instance, the relief 210 has a generally cylindrical profile and the keepers 212 each have a generally semicircular profile with notches 214 arranged to abut the harness 189. Here, engagement between the keepers 212 and the relief 210 may create a "compression fit" to limt movement of the harness 189. In some versions, one or mor eof the notches 214 may be provided with teeth 216 or other formations to promote retention of the harness 189. In some versiosn, the teeth 216 may be realized as a "thread" configuration, such as with a helical arrangement of teeth 216. The keepers 212 and the relief 210 of the retainer assembly 208 help prevent movement of the harness 189 relative to the shielding enclosure 190 and, thus, relative to the connector 191 attached to the reference detector board 187.

In this application, including the definitions below, the term "controller" may be replaced with the term "circuit." The term "controller" may refer to, be part of, or include: an Application Specific Integrated Circuit (ASIC); a digital, analog, or mixed analog/digital discrete circuit; a digital, analog, or mixed analog/digital integrated circuit; a combinational logic circuit; a field programmable gate array (FPGA); a processor circuit (shared, dedicated, or group) that executes code; a memory circuit (shared, dedicated, or group) that stores code executed by the processor circuit; other suitable hardware components that provide the described functionality; or a combination of some or all of the above, such as in a system-on-chip.

The one or more controller(s) may include one or more interface circuits. In some examples, the interface circuit(s) may implement wired or wireless interfaces that connect to a local area network (LAN) or a wireless personal area network (WPAN). Examples of a LAN are Institute of Electrical and Electronics Engineers (IEEE) Standard 802.11-2016 (also known as the WIFI wireless networking standard) and IEEE Standard 802.3-2015 (also known as the ETHERNET wired networking standard). Examples of a WPAN are the BLUETOOTH wireless networking standard from the Bluetooth Special Interest Group and IEEE Standard 802.15.4.

The one or more controllers may communicate with other controllers using the interface circuit(s). Although the controller may be depicted in the present disclosure as logically communicating directly with other controllers, in various configurations the controller may actually communicate via a communications system. The communications system includes physical and/or virtual networking equipment such as hubs, switches, routers, and gateways. In some configurations, the communications system connects to or traverses a wide area network (WAN) such as the Internet. For example, the communications system may include multiple LANs connected to each other over the Internet or point-to-point leased lines using technologies including Multiprotocol Label Switching (MPLS) and virtual private networks (VPNs).

In various configurations, the functionality of the controller may be distributed among multiple controllers that are connected via the communications system. For example, multiple controllers may implement the same functionality distributed by a load balancing system. In a further example, the functionality of the controller may be split between a server (also known as remote, or cloud) controller and a client (or, user) controller.

Some or all hardware features of a controller may be defined using a language for hardware description, such as IEEE Standard 1364-2005 (commonly called "Verilog") and IEEE Standard 10182-2008 (commonly called "VHDL"). The hardware description language may be used to manufacture and/or program a hardware circuit. In some configurations, some or all features of a controller may be defined by a language, such as IEEE 1666-2005 (commonly called "SystemC"), that encompasses both code, as described below, and hardware description.

The various controller programs may be stored on a memory circuit. The term memory circuit is a subset of the term computer-readable medium. The term computer-readable medium, as used herein, does not encompass transitory electrical or electromagnetic signals propagating through a medium (such as on a carrier wave); the term computer-readable medium may therefore be considered tangible and non-transitory. Non-limiting examples of a non-transitory computer-readable medium are nonvolatile memory circuits (such as a flash memory circuit, an erasable programmable read-only memory circuit, or a mask read-only memory circuit), volatile memory circuits (such as a static random access memory circuit or a dynamic random access memory circuit), magnetic storage media (such as an analog or digital magnetic tape or a hard disk drive), and optical storage media (such as a CD, a DVD, or a Blu-ray Disc).

The apparatuses and methods described in this application may be partially or fully implemented by a special purpose computer created by configuring a general purpose computer to execute one or more particular functions embodied in computer programs. The functional blocks and flowchart elements described above serve as software specifications, which can be translated into the computer programs by the routine work of a skilled technician or programmer.

The computer programs include processor-executable instructions that are stored on at least one non-transitory computer-readable medium. The computer programs may also include or rely on stored data. The computer programs may encompass a basic input/output system (BIOS) that interacts with hardware of the special purpose computer, device drivers that interact with particular devices of the special purpose computer, one or more operating systems, user applications, background services, background applications, etc.

The computer programs may include: (i) descriptive text to be parsed, such as HTML (hypertext markup language), XML (extensible markup language), or JSON (JavaScript Object Notation), (ii) assembly code, (iii) object code generated from source code by a compiler, (iv) source code for execution by an interpreter, (v) source code for compilation and execution by a just-in-time compiler, etc. As examples only, source code may be written using syntax from languages including C, C++, C#, Objective C, Swift, Haskell, Go, SQL, R, Lisp, Java^{®}, Fortran, Perl, Pascal, Curl, OCaml, JavaScript^{®}, HTML5 (Hypertext Markup Language 5th revision), Ada, ASP (Active Server Pages), PHP (PHP: Hypertext Preprocessor), Scala, Eiffel, Smalltalk, Erlang, Ruby, Flash^{®}, Visual Basic^{®}, Lua, MATLAB, SENSORLINK, and Python^{®}.

## Claims

1. An x-ray CT system (100), comprising:
a gantry (40) with a rotor (41) arranged for rotation about an axis;
an x-ray source (43) supported on the rotor (41) and configured to generate x-rays;
an x-ray detector (45) supported on the rotor (41);
a reference detector assembly (166) operatively attached to the x-ray source (43) for measuring flux of photons generated by the x-ray source (43), the reference detector assembly (166) including:
a tungsten shield (180) defining an aperture (188),
an x-ray sensitive element (182) supported adjacent to the aperture (188) of the tungsten shield (180) and configured to generate a reference output in response to x-rays generated by the x-ray source (43) passing through the aperture (188),
a photodiode (184) supported adjacent to the x-ray sensitive element (182) and configured to receive the reference output from the x-ray sensitive element (182), and
a reference detector controller (186) in communication with the photodiode (184) and configured to generate a reference signal based on the reference output from the x-ray sensitive element (182); and
a controller (46) including a memory (104) and a processor (102) coupled to the memory (104) and configured with processor-executable instructions to perform tomographic reconstruction of image data received from the x-ray detector (45) and normalized based on the reference signal from the reference detector controller (186).

2. The x-ray CT system (100) of claim 1, wherein the reference detector assembly (166) further includes a shielding enclosure (190); and
wherein the tungsten shield (180) is operatively attached to the shielding enclosure (190).

3. The x-ray CT system (100) of claim 2, wherein at least a portion of the shielding enclosure (190) is formed from at least one of leaded bronze and tungsten.

4. The x-ray CT system (100) of claim 2, wherein at least a portion of the shielding enclosure (190) is formed using an additive manufacturing process, and wherein, optionally, the additive manufacturing process comprises selective laser melting or laser sintering.

5. The x-ray CT system (100) of claim 2, wherein one of the shielding enclosure (190) and the tungsten shield (180) defines an interior (192).

6. The x-ray CT system (100) of claim 5, further comprising a reference detector board (187) disposed within the interior (192) and supporting the photodiode (184), and wherein, optionally, the x-ray sensitive element (182) is supported within the interior (192) arranged between the tungsten shield (180) and the reference detector board (187); and further optionally at least one of:
the reference detector controller (186) is supported on the reference detector board (187) at a location spaced from the aperture (188); and
the reference detector assembly (166) further includes an insulator supported within the interior (192) adjacent to the reference detector board (187); and
the reference detector assembly (166) further includes a heat transfer pad supported within the interior (192) adjacent to the reference detector board (187).

7. The x-ray CT system (100) of claim 6, further comprising:
a harness (189) coupled to the reference detector board (187) via a connector (191); and
a retainer assembly (208) to limit relative movement between the harness (189) and the shielding enclosure (190), wherein, optionally, the retainer assembly (208) includes a relief (210) defined in the shielding enclosure (190) and shaped to receive a pair of keepers (212) each shaped to engage and compress against a portion of the harness (189), and wherein, further optionally, the pair of keepers (212) each define a notch (214) arranged to abut the harness (189).

8. The x-ray CT system (100) of claim 2, wherein the shielding enclosure (190) includes a first enclosure plate (190) and a second enclosure plate (190), the first enclosure plate (190) being coupled to the second enclosure plate (190) with the tungsten shield (180) supported between the first enclosure plate (190) and the second enclosure plate (190).

9. The x-ray CT system (100) of claim 8, wherein one of the first enclosure plate (190) and the second enclosure plate (190) defines an interior (192); and
wherein the reference detector assembly (166) further includes a reference detector board (187) disposed within the interior (192) and supporting the photodiode (184), with the x-ray sensitive element (182) arranged between the tungsten shield (180) and the reference detector board (187), and wherein, optionally, at least one of the first enclosure plate (190) and the second enclosure plate (190) defines a seat (197) shaped to receive the reference detector board (187) within the interior (192).

10. The x-ray CT system (100) of claim 9, wherein one of the first enclosure plate (190) and the second enclosure plate (190) defines a window (200); and
wherein the reference detector assembly (166) further includes an auxiliary tungsten shield (202) defining an auxiliary aperture (204), the auxiliary tungsten shield (202) being secured to the window (200) with the auxiliary aperture (204) in alignment with the aperture (188) of the tungsten shield (180) to permit x-rays generated by the x-ray source (43) to pass through the auxiliary aperture (204) and through the aperture (188) towards the x-ray sensitive element (182).

11. The x-ray CT system (100) of claim 1, wherein the reference output generated by the x-ray sensitive element (182) is visible light, and wherein the photodiode (184) is configured to sense the visible light outputted by the x-ray sensitive element (182), and wherein, optionally:
the x-ray sensitive element (182) comprises a crystal scintillator.

12. The x-ray CT system (100) of claim 1, wherein the x-ray detector (45) comprises an array of x-ray detector modules (107); and
wherein the x-ray source (43) is further configured to generate a fan beam of x-rays.

13. The x-ray CT system (100) of claim **1,** further comprising a second a reference detector assembly (166) operatively attached to the x-ray source (43) in spaced relation from the reference detector assembly (166) for measuring flux of photons generated by the x-ray source (43), the second reference detector assembly (166) including:
a second tungsten shield (202) defining a second aperture (204),
a second x-ray sensitive element (182) supported adjacent to the second aperture (204) of the second tungsten shield (202) and configured to generate a second reference output in response to x-rays generated by the x-ray source (43) passing through the second aperture (204),
a second photodiode (184) supported adjacent to the second x-ray sensitive element (182) to receive the second reference output from the x-ray sensitive element (182), and
a second reference detector controller (186) in communication with the second photodiode (184) to generate a second reference signal based on the second reference output from the second x-ray sensitive element (182).

14. The x-ray CT system (100) of claim 13, wherein the controller (46) is further configured to perform tomographic reconstruction with image data received from the x-ray detector (45) normalized based on one or more of the reference signal from the reference detector controller (186) and the second reference signal from the second reference detector controller (186).

15. The x-ray CT system (100) of claim 13, wherein the x-ray source (43) is configured to generate x-rays based at least partially on one or more of the reference signal from the reference detector controller (186) and the second reference signal from the second reference detector controller (186).

## Patentansprüche

1. Röntgen-CT-System (100), umfassend:
eine Gantry (40) mit einem Rotor (41), der zur Drehung um eine Achse angeordnet ist;
eine Röntgenquelle (43), die auf dem Rotor (41) gelagert und zur Erzeugung von Röntgenstrahlen ausgelegt ist;
einen auf dem Rotor (41) gelagerten Röntgendetektor (45);
eine Referenzdetektoranordnung (166), die funktionsfähig mit der Röntgenquelle (43) verbunden ist, um den von der Röntgenquelle (43) erzeugten Photonenfluss zu messen, wobei die Referenzdetektoranordnung (166) umfasst:
eine Wolframabschirmung (180), die eine Öffnung (188) definiert,
ein röntgenempfindliches Element (182), das benachbart zur Öffnung (188) der Wolframabschirmung (180) gelagert und so ausgelegt ist, dass es als Reaktion auf von der Röntgenquelle (43) erzeugte Röntgenstrahlen, die durch die Öffnung (188) hindurchtreten, eine Referenzausgabe erzeugt,
eine Fotodiode (184), die benachbart zum röntgenempfindlichen Element (182) gelagert und so ausgelegt ist, dass sie die Referenzausgabe vom röntgenempfindlichen Element (182) empfängt, und
eine Referenzdetektor-Steuerung (186), die mit der Fotodiode (184) in kommunikativer Verbindung steht und so ausgelegt ist, dass sie bezogen auf die Referenzausgabe vom röntgenempfindlichen Element (182) ein Referenzsignal erzeugt; sowie
eine Steuerung (46) mit einem Speicher (104) und einem mit dem Speicher (104) gekoppelten Prozessor (102), der mit prozessorausführbaren Befehlen ausgestattet ist, um eine tomografische Rekonstruktion von Bilddaten durchzuführen, die vom Röntgendetektor (45) empfangen und bezogen auf das Referenzsignal von der Referenzdetektor-Steuerung (186) normalisiert wurden.

2. Röntgen-CT-System (100) nach Anspruch 1, wobei die Referenzdetektoranordnung (166) ferner ein Abschirmgehäuse (190) umfasst; und
wobei die Wolframabschirmung (180) funktionsfähig an dem Abschirmgehäuse (190) befestigt ist.

3. Röntgen-CT-System (100) nach Anspruch 2, wobei zumindest ein Teil des Abschirmgehäuses (190) aus mindestens einem der Werkstoffe Bleibronze und Wolfram gebildet ist.

4. Röntgen-CT-System (100) nach Anspruch 2, wobei zumindest ein Teil des Abschirmgehäuses (190) unter Verwendung eines additiven Fertigungsverfahrens hergestellt ist und wobei optional das additive Fertigungsverfahren selektives Laserschmelzen oder Lasersintern umfasst.

5. Röntgen-CT-System (100) nach Anspruch 2, wobei entweder das Abschirmgehäuse (190) oder die Wolframabschirmung (180) einen Innenraum (192) definiert.
T:\60\P-2156\Anlagen Klage

6. Röntgen-CT-System (100) nach Anspruch 5, ferner umfassend eine Referenzdetektorplatine (187), die innerhalb des Innenraums (192) angeordnet ist und die Fotodiode (184) trägt, und wobei optional das röntgenempfindliche Element (182) innerhalb des Innenraums (192) zwischen der Wolframabschirmung (180) und der Referenzdetektorplatine (187) gelagert ist; und ferner optional mindestens eines der folgenden Merkmale vorliegt:
die Referenzdetektor-Steuerung (186) ist auf der Referenzdetektorplatine (187) an einer von der Öffnung (188) beabstandeten Stelle gelagert; und
die Referenzdetektoranordnung (166) umfasst ferner einen Isolator, der im Innenraum (192) angrenzend zur Referenzdetektorplatine (187) gelagert ist; und
die Referenzdetektoranordnung (166) umfasst ferner ein Wärmeübertragungskissen, das im Innenraum (192) angrenzend an die Referenzdetektorplatine (187) gelagert ist.

7. Röntgen-CT-System (100) nach Anspruch 6, ferner umfassend:
einen Kabelbaum (189), der über einen Steckverbinder (191) mit der Referenzdetektorplatine (187) verbunden ist; und
eine Halteanordnung (208) zur Begrenzung einer Relativbewegung zwischen dem Kabelbaum (189) und dem Abschirmgehäuse (190), wobei optional die Halteanordnung (208) eine im Abschirmgehäuse (190) ausgebildete Ausnehmung (210) umfasst, die so geformt ist, dass sie ein Paar Halteelemente (212) aufnimmt, die jeweils so geformt sind, dass sie an einem Abschnitt des Kabelbaums (189) angreifen und diesen zusammendrücken, und wobei ferner optional die beiden Halteelemente (212) jeweils eine Kerbe (214) aufweisen, die so angeordnet ist, dass sie an dem Kabelbaum (189) anliegt.

8. Röntgen-CT-System (100) nach Anspruch 2, wobei das Abschirmgehäuse (190) eine erste Gehäuseplatte (190) und eine zweite Gehäuseplatte (190) umfasst, wobei die erste Gehäuseplatte (190) mit der zweiten Gehäuseplatte (190) verbunden ist und die Wolframabschirmung (180) zwischen der ersten Gehäuseplatte (190) und der zweiten Gehäuseplatte (190) gelagert ist.

9. Röntgen-CT-System (100) nach Anspruch 8, wobei entweder die erste Gehäuseplatte (190) oder die zweite Gehäuseplatte (190) einen Innenraum (192) definiert; und
wobei die Referenzdetektoranordnung (166) ferner eine Referenzdetektorplatine (187) umfasst, die innerhalb des Innenraums (192) angeordnet ist und die Fotodiode (184) trägt, wobei das röntgenempfindliche Element (182) zwischen der Wolframabschirmung (180) und der Referenzdetektorplatine (187) angeordnet ist, und wobei optional mindestens eine der ersten Gehäuseplatte (190) und der zweiten Gehäuseplatte (190) eine Aufnahme (197) bildet, die so geformt ist, dass sie die Referenzdetektorplatine (187) im Innenraum (192) aufnimmt.

10. Röntgen-CT-System (100) nach Anspruch 9, wobei entweder die erste Gehäuseplatte (190) oder die zweite Gehäuseplatte (190) ein Fenster (200) definiert; und
wobei die Referenzdetektoranordnung (166) ferner eine zusätzliche Wolframabschirmung (202) umfasst, die eine zusätzliche Öffnung (204) definiert, wobei die zusätzliche Wolframabschirmung (202) an dem Fenster (200) befestigt ist, wobei die zusätzliche Öffnung (204) in Ausrichtung mit der Öffnung (188) der Wolframabschirmung (180) an dem Fenster (200) befestigt ist, sodass von der Röntgenquelle (43) erzeugte Röntgenstrahlen durch die zusätzliche Öffnung (204) und durch die Öffnung (188) in Richtung des röntgenempfindlichen Elements (182) gelangen können.

11. Röntgen-CT-System (100) nach Anspruch 1, wobei die vom röntgenempfindlichen Element (182) erzeugte Referenzausgabe sichtbares Licht ist und wobei die Fotodiode (184) so ausgelegt ist, dass sie das vom röntgenempfindlichen Element (182) ausgegebene sichtbare Licht erfasst, und wobei optional:
das röntgenempfindliche Element (182) einen Kristallszintillator umfasst.

12. Röntgen-CT-System (100) nach Anspruch 1, wobei der Röntgendetektor (45) eine Anordnung von Röntgendetektormodulen (107) umfasst; und
wobei die Röntgenquelle (43) ferner so ausgelegt ist, dass sie einen Fächerstrahl aus Röntgenstrahlen erzeugt.

13. Röntgen-CT-System (100) nach Anspruch 1, das ferner eine zweite Referenzdetektoranordnung (166) umfasst, die funktionsfähig an der Röntgenquelle (43) in einem Abstand zur Referenzdetektoranordnung (166) angebracht ist, um den von der Röntgenquelle (43) erzeugten Photonenfluss zu messen, wobei die zweite Referenzdetektoranordnung (166) umfasst:
eine zweite Wolframabschirmung (202), die eine zweite Öffnung (204) definiert,
ein zweites röntgenempfindliches Element (182), das angrenzend zur zweiten Öffnung (204) der zweiten Wolframabschirmung (202) gelagert und so ausgelegt ist, dass es eine zweite Referenzausgabe als Reaktion auf von der Röntgenquelle (43) erzeugte Röntgenstrahlen erzeugt, die durch die zweite Öffnung (204) hindurchtreten,
eine zweite Fotodiode (184), die angrenzend zum zweiten röntgenempfindlichen Element (182) gelagert ist, um die zweite Referenzausgabe vom röntgenempfindlichen Element (182) zu empfangen, und
eine zweite Referenzdetektor-Steuerung (186), die mit der zweiten Fotodiode (184) in kommunikativer Verbindung steht, um bezogen auf die zweite Referenzausgabe des zweiten röntgenempfindlichen Elements (182) ein zweites Referenzsignal zu erzeugen.

14. Röntgen-CT-System (100) nach Anspruch 13, wobei die Steuerung (46) ferner so konfiguriert ist, dass sie eine tomografische Rekonstruktion mit Bilddaten durchführt, die vom Röntgendetektor (45) empfangen und bezogen auf das Referenzsignal von der Referenzdetektor-Steuerung (186) und/oder das zweite Referenzsignal von der zweiten Referenzdetektor-Steuerung (186) normiert wurden.

15. Röntgen-CT-System (100) nach Anspruch 13, wobei die Röntgenquelle (43) so ausgelegt ist, dass sie Röntgenstrahlen zumindest teilweise bezogen auf das Referenzsignal von der Referenzdetektor-Steuerung (186) und/oder das zweite Referenzsignal von der zweiten Referenzdetektor-Steuerung (186) erzeugt.

## Revendications

1. Système de tomodensitométrie à rayons X (100) comprenant:
un portique (40) équipé d'un rotor (41) disposé pour tourner autour d'un axe;
une source de rayons X (43) montée sur le rotor (41) et conçue pour générer des rayons X;
un détecteur de rayons X (45) monté sur le rotor (41);
un ensemble détecteur de référence (166) fixé fonctionnellement à la source de rayons X (43) afin de mesurer le flux de photons générés par la source de rayons X (43), l'ensemble détecteur de référence (166) comprenant:
un blindage en tungstène (180) délimitant une ouverture (188),
un élément sensible aux rayons X (182) monté à proximité de l'ouverture (188) du blindage en tungstène (180) et conçu pour générer un signal de sortie de référence en réponse aux rayons X émis par la source de rayons X (43) et traversant l'ouverture (188),
une photodiode (184) adjacent à l'élément sensible aux rayons X (182) et conçue pour recevoir le signal de référence provenant de l'élément sensible aux rayons X (182), et
un organe de commande de détecteur de référence (186) en communication avec la photodiode (184) et configuré pour générer un signal de référence à partir du signal de sortie de référence provenant de l'élément sensible aux rayons X (182); et
un contrôleur (46) comprenant une mémoire (104) et un processeur (102) couplé à la mémoire (104) et configuré avec des instructions exécutables par processeur pour effectuer une reconstruction tomographique des données d'image reçues en provenance du détecteur de rayons X (45) et normalisées sur la base du signal de référence provenant du contrôleur du détecteur de référence (186).

2. Système de tomodensitométrie à rayons X (100) selon la revendication 1, dans lequel l'ensemble détecteur de référence (166) comprend en outre une enceinte de blindage (190); et
dans lequel le blindage en tungstène (180) est fixé fonctionnellement à l'enceinte de blindage (190).

3. Système de tomodensitométrie à rayons X (100) selon la revendication 2, dans lequel est constitué d'au moins l'un parmi le bronze au plomb ou le tungstène.

4. Système de tomodensitométrie à rayons X (100) selon la revendication 2, dans lequel au moins une partie de l'enceinte de blindage (190) est réalisée à l'aide d'un procédé de fabrication additive, et dans lequel, éventuellement, le procédé de fabrication additive comprend la fusion sélective au laser ou le frittage au laser.

5. Système de tomodensitométrie à rayons X (100) selon la revendication 2, dans lequel l'une parmi l'enceinte de blindage (190) et le blindage en tungstène (180) délimite un espace intérieur (192).

6. Système de tomodensitométrie à rayons X (100) selon la revendication 5, comprenant en outre une carte de détection de référence (187) disposée dans l'espace intérieur (192) et supportant la photodiode (184), et dans lequel, éventuellement, l'élément sensible aux rayons X (182) est supporté dans l'espace intérieur (192), étant disposé entre le blindage en tungstène (180) et la carte de détection de référence (187); et, éventuellement également, au moins l'un parmi:
le contrôleur du détecteur de référence (186) est monté sur la carte de détecteur de référence (187) à un emplacement à l'écart de l'ouverture (188); et
l'ensemble détecteur de référence (166) comprend en outre un isolant logé dans l'espace intérieur (192), à proximité de la carte du détecteur de référence (187); et
l'ensemble détecteur de référence (166) comprend en outre un tampon de transfert thermique logé dans l'espace intérieur (192), à proximité de la carte de détection de référence (187).

7. Système de tomodensitométrie à rayons X (100) selon la revendication 6, comprenant en outre:
un faisceau (189) relié à la carte de détection de référence (187) par l'intermédiaire d'un connecteur (191); et
un ensemble de retenue (208) destiné à limiter le mouvement relatif entre le faisceau (189) et l'enceinte de blindage (190), dans lequel, éventuellement, l'ensemble de retenue (208) comprend un évidement (210) défini dans l'enceinte de blindage (190) et formé pour recevoir une paire de gâchettes (212), chacune étant formée pour venir en prise avec une partie du faisceau (189) et exercer une pression contre celle-ci, et dans lequel, en outre éventuellement, la paire de gâchettes (212) définit chacun une encoche (214) agencée pour venir en butée contre le faisceau (189).

8. Système de tomodensitométrie à rayons X (100) selon la revendication 2, dans lequel l'enceinte de blindage (190) comprend une première plaque d'enceinte (190) et une deuxième plaque d'enceinte (190), la première plaque d'enceinte (190) étant reliée à la deuxième plaque d'enceinte (190), le blindage en tungstène (180) étant supporté entre la première plaque d'enceinte (190) et la deuxième plaque d'enceinte (190).

9. Système de tomodensitométrie à rayons X (100) selon la revendication 8, dans lequel l'une des première plaque de boîtier (190) et deuxième plaque de boîtier (190) délimite un espace intérieur (192); et
dans lequel l'ensemble détecteur de référence (166) comprend en outre une carte de détecteur de référence (187) disposée dans l'espace intérieur (192) et supportant la photodiode (184), l'élément sensible aux rayons X (182) étant disposé entre le blindage en tungstène (180) et la carte de détecteur de référence (187), et dans lequel, éventuellement, au moins l'une parmi la première plaque d'enceinte (190) et la deuxième plaque d'enceinte (190) délimite un logement (197) formé de manière à recevoir la carte de détecteur de référence (187) à l'intérieur de l'espace intérieur (192).

10. Système de tomodensitométrie à rayons X (100) selon la revendication 9, dans lequel l'une des première plaque de boîtier (190) et la deuxième plaque de boîtier (190) délimitent une fenêtre (200); et
dans lequel l'ensemble détecteur de référence (166) comprend en outre un blindage auxiliaire en tungstène (202) délimitant une ouverture auxiliaire (204), le blindage auxiliaire en tungstène (202) étant fixé à la fenêtre (200), l'ouverture auxiliaire (204) étant alignée avec l'ouverture (188) du blindage en tungstène (180) afin de permettre aux rayons X générés par la source de rayons X (43) de traverser l'ouverture auxiliaire (204) et traverser l'ouverture (188) en direction de l'élément sensible aux rayons X (182).

11. Système de tomodensitométrie à rayons X (100) selon la revendication 1, dans lequel le signal de sortie de référence généré par l'élément sensible aux rayons X (182) est de la lumière visible, et dans lequel la photodiode (184) est conçue pour détecter la lumière visible émise par l'élément sensible aux rayons X (182), et dans lequel, éventuellement:
l'élément sensible aux rayons X (182) comprend un scintillateur cristallin.

12. Système de tomodensitométrie à rayons X (100) selon la revendication 1, dans lequel le détecteur de rayons X (45) comprend un réseau de modules de détection de rayons X (107); et
dans lequel la source de rayons X (43) est en outre conçue pour générer un faisceau en éventail de rayons X.

13. Système de tomodensitométrie à rayons X (100) selon la revendication 1, comprenant en outre un deuxième ensemble détecteur de référence (166) relié fonctionnellement à la source de rayons X (43), à une certaine distance de l'ensemble détecteur de référence (166), afin de mesurer le flux de photons générés par la source de rayons X (43), le deuxième ensemble détecteur de référence (166) comprenant:
un deuxième blindage en tungstène (202) délimitant une deuxième ouverture (204),
un deuxième élément sensible aux rayons X (182) monté à proximité de la deuxième ouverture (204) du deuxième blindage en tungstène (202) et conçu pour générer un deuxième signal de sortie de référence en réponse aux rayons X émis par la source de rayons X (43) et traversant la deuxième ouverture (204),
une deuxième photodiode (184) adjacent au deuxième élément sensible aux rayons X (182) et conçue pour recevoir le deuxième signal de référence provenant de l'élément sensible aux rayons X (182), et
un deuxième organe de commande de détecteur de référence (186) en communication avec la deuxième photodiode (184) pour générer un deuxième signal de référence à partir du deuxième signal de sortie de référence provenant du deuxième élément sensible aux rayons X (182).

14. Système de tomodensitométrie à rayons X (100) selon la revendication 13, dans lequel le contrôleur (46) est en outre configuré pour effectuer une reconstruction tomographique à partir des données d'image reçues en provenance du détecteur de rayons X (45), normalisées sur la base d'un ou plusieurs parmi le signal de référence provenant du contrôleur de détecteur de référence (186) et le deuxième signal de référence provenant du deuxième contrôleur du détecteur de référence (186).

15. Système de tomodensitométrie à rayons X (100) selon la revendication 13, dans lequel la source de rayons X (43) est configurée pour générer des rayons X en fonction, au moins en partie, d'un ou plusieurs parmi le signal de référence provenant du contrôleur de détecteur de référence (186) et le deuxième signal de référence provenant du deuxième contrôleur de détecteur de référence (186).
